# EUROPEAN PATENT SPECIFICATION

(11) **EP 4 137 045 B1**
(45) Date of publication and mention of the grant of the patent: **27.08.2025**
(21) Application number: 21808974.6
(22) Date of filing: 12.04.2021
(51) Int. Cl.: A61B 5/0537, A61B 5/00, A61B 5/05

(54) **BODY COMPOSITION TEST METHOD AND DEVICE**
KÖRPERZUSAMMENSETZUNGSTESTVERFAHREN UND -VORRICHTUNG
PROCÉDÉ ET DISPOSITIF DE TEST DE COMPOSITION CORPORELLE

(30) Priority: 19.05.2020 CN 202010423255
(43) Date of publication of application: 22.02.2023
(73) Proprietor: Huawei Technologies Co., Ltd., Longgang District, Shenzhen, Guangdong 518129 (CN)
(72) Inventor: ZHAO, Shuai, Shenzhen, Guangdong 518129 (CN); YANG, Bin, Shenzhen, Guangdong 518129 (CN); REN, Huichao, Shenzhen, Guangdong 518129 (CN); XIONG, Hao, Shenzhen, Guangdong 518129 (CN)
(74) Representative: Pfenning, Meinig & Partner mbB
(86) International application number: PCT/CN2021/086699
(87) International publication number: WO 2021/233011

(56) References cited:
- CN-A- 1 387 821
- CN-A- 105 550 717
- CN-A- 105 708 459
- CN-A- 106 580 322
- CN-A- 106 768 245
- CN-A- 107 242 871
- CN-A- 107 731 302
- CN-A- 109 243 611
- US-A1- 2004 054 298
- US-A1- 2009 018 464
- US-A1- 2010 094 157
- US-A1- 2018 296 136

## Description

### TECHNICAL FIELD

The present invention relates to the field of body composition detection technologies, and in particular, to a body composition detection method and device, and a computer-readable storage medium.

### BACKGROUND

Currently, a most commonly used method for body composition detection is bioimpedance analysis (bioimpedance analysis, BIA for short). When a weak current passes through a human body, composition such as fat and muscle in the human body has different electrical conductivity, and generates different human body impedances. Therefore, content of various types of body composition can be calculated by using a measured impedance in combination with information such as gender, age, height, and weight. A BIA-based body fat scale includes a four-electrode body fat scale or an eight-electrode body fat scale. When a user needs to calculate segmental body composition, the eight-electrode body fat scale is required to calculate the segmental body composition. In addition, the eight-electrode body fat scale has a high requirement on a two-arm posture and a handle holding posture of the user. In a process of detecting body composition by using the body fat scale, because a handle is not tightly held and a posture is incorrect, impedance measurement is inaccurate, and a body fat rate measurement error is easily caused, resulting in problems such as a user identity identification error and a large error in a body composition detection result.

CN 107242871 A addresses a method for automatically judging whether to use four-electrode measurement or eight-electrode measurement in body impedance measurement. CN 107731302 A proposes an information management method that comprises: a background server receives reference impedance information sent by a weight detecting device; the background server matches the reference impedance information with historical impedance information of all user account numbers corresponding to the weight detecting device; and, when matching is done successfully, the background server stores the reference impedance information and the user account number matched successfully in an association manner. US 2004/054298 A1 describes body impedance measurement apparatus including a measuring unit for supplying a weak current through the body of a subject from current-carrying electrodes attached to the body and measuring a voltage generated by the current with measuring electrodes attached to the body, and a calculating unit for calculating an impedance of the body from the value of the current supplied through the body and the value of the voltage measured. US 2009/018464 A1 concerns a body composition measuring device that measures body composition by bioelectricity impedance measurement, and body composition is measured from a value of bioelectricity impedance measurement using a plurality of regressions.

### SUMMARY

In view of this, the present invention provides a body composition detection method and an electronic device according to the independent claims.
Said body composition detection method and said electronic device can generate, while identifying user identity, body composition of a current user in different detection modes based on obtained detection data in the different detection modes. In this way, accuracy and efficiency of body composition detection are improved. Particular embodiments are defined in the dependent claims.

According to one aspect, an embodiment of the present invention provides a body composition detection method performed by an electronic device including a body fat scale, wherein the body fat scale includes a body fat scale compatible with a four-electrode mode and an eight-electrode mode, the method including:
detecting a detection mode used by a current user wherein the detection mode comprises an eight-electrode mode and a four-electrode mode;
performing identity identification on the current user based on the detection mode, a measured weight and at least one impedance of the current user, and obtained user data of a plurality of users, to generate a user identification result; and
generating body composition of the current user in different detection modes based on obtained detection data in the different detection modes.

In a possible implementation, the detection mode includes the eight-electrode mode, the at least one impedance includes a plurality of segmental impedances, the plurality of segmental impedances include a first impedance, and the user data includes a historical weight, a historical first impedance, a historical body fat rate, and user attribute information.

The performing identity identification on the current user based on the detection mode, a measured weight and at least one impedance of the current user, and obtained user data of a plurality of users, to generate a user identification result includes:
determining a first user set based on the measured weight of the current user and the historical weights of the plurality of users who include the current user;
determining a second user set based on the first user set, the measured weight of the current user, the first impedance, and the historical first impedance, the historical body fat rate, and the user attribute information of each user; and
performing identity identification on the current user based on the second user set, to generate the user identification result.

The performing identity identification on the current user based on the second user set, to generate the user identification result includes:
selecting, from the second user set, a user corresponding to a minimum third change rate, and determining the user corresponding to the minimum third change rate as the current user.

The detection mode includes the four-electrode mode.

After the selecting, from the second user set, a user corresponding to a minimum third change rate, and determining the user corresponding to the minimum third change rate as the current user, the method further includes:
obtaining a current measurement time point, and determining whether a time interval between a measurement time point at which an eight-electrode mode is used for detection last time and the current measurement time point is less than a preset time period; and
if it is determined that the time interval between the measurement time point at which the eight-electrode mode is used last time and the current measurement time point is greater than the preset time period, entering a four-electrode common mode, and continuing to perform the step of calculating total body composition of the current user based on the weight of the current user, the first impedance, and the obtained user attribute information of the current user; or
if it is determined that the time interval between the measurement time point at which the eight-electrode mode is used last time and the current measurement time point is less than the preset time period, entering a four-electrode expansion mode, and continuing to perform the step of calculating total body composition and segmental body composition of the current user based on the weight of the current user, the first impedance, the obtained impedance other than the first impedance of the current user, and the obtained user attribute information of the current user.

According to a second aspect, an embodiment of the present invention provides a body composition detection device. The device includes:
detecting a detection mode used by a current user;
performing identity identification on the current user based on the detection mode, a measured weight and at least one impedance of the current user, and obtained user data of a plurality of users, to generate a user identification result; and
generating body composition of the current user in different detection modes based on obtained detection data in the different detection modes.

The performing identity identification on the current user based on the detection mode, a measured weight and at least one impedance of the current user, and obtained user data of a plurality of users, to generate a user identification result includes:
determining a first user set based on the measured weight of the current user and the historical weights of the plurality of users who include the current user;
determining a second user set based on the first user set, the measured weight of the current user, the first impedance, and the historical first impedance, the historical body fat rate, and the user attribute information of each user; and
performing identity identification on the current user based on the second user set, to generate the user identification result.

The performing identity identification on the current user based on the second user set, to generate the user identification result includes:
selecting, from the second user set, a user corresponding to a minimum third change rate, and determining the user corresponding to the minimum third change rate as the current user.

When the detection mode includes the four-electrode mode:
After the selecting, from the second user set, a user corresponding to a minimum third change rate, and determining the user corresponding to the minimum third change rate as the current user, the device further includes:
obtaining a current measurement time point, and determining whether a time interval between a measurement time point at which an eight-electrode mode is used for detection last time and the current measurement time point is less than a preset time period; and
if it is determined that the time interval between the measurement time point at which the eight-electrode mode is used last time and the current measurement time point is greater than the preset time period, entering a four-electrode common mode, and continuing to perform the step of calculating total body composition of the current user based on the weight of the current user, the first impedance, and the obtained user attribute information of the current user; or
if it is determined that the time interval between the measurement time point at which the eight-electrode mode is used last time and the current measurement time point is less than the preset time period, entering a four-electrode expansion mode, and continuing to perform the step of calculating total body composition and segmental body composition of the current user based on the weight of the current user, the first impedance, the obtained impedance other than the first impedance of the current user, and the obtained user attribute information of the current user. (next paragraph: [0067])

According to a related third aspect, a computer-readable storage medium is provided. The computer-readable storage medium includes a stored program, and when the program runs, a
device in which the computer-readable storage medium is located is controlled to perform the foregoing body composition detection method.

In the technical solutions provided in embodiments of the present invention, a detection mode used by a current user is detected. Identity identification is performed on the current user based on the detection mode, a measured weight and at least one impedance of the current user, and obtained user data of a plurality of users, to generate a user identification result. Body composition of the current user in different detection modes is generated based on obtained detection data in the different detection modes. In this way, accuracy and efficiency of body composition detection are improved.

### BRIEF DESCRIPTION OF DRAWINGS

FIG. 1 is an architectural diagram of a body composition detection system according to an embodiment of the present invention;
FIG. 2 is a schematic diagram of a structure of a body fat scale in a four-electrode mode according to an embodiment of the present invention;
FIG. 3 is a schematic diagram of detecting body composition of a user in a four-electrode mode according to an embodiment of the present invention;
FIG. 4 is a schematic diagram of a structure of a body fat scale in an eight-electrode mode according to an embodiment of the present invention;
FIG. 5 is a schematic diagram of detecting body composition of a user in an eight-electrode mode according to an embodiment of the present invention;
FIG. 6 is a block diagram of a structure of a detection mode according to an embodiment of the present invention;
FIG. 7A, FIG. 7B, and FIG. 7C are a flowchart of a body composition detection method according to an embodiment of the present invention;
FIG. 8A, FIG. 8B, and FIG. 8C are a flowchart of another body composition detection method according to an embodiment of the present invention;
FIG. 9 is a flowchart of still another body composition detection method according to an embodiment of the present invention;
FIG. 10A and FIG. 10B are a flowchart of user identity identification according to an embodiment of the present invention;
FIG. 11 is a schematic diagram of a structure of user data according to an embodiment of the present invention;
FIG. 12 is a schematic diagram of a display interface according to an embodiment of the present invention;
FIG. 13 is a schematic diagram of a display interface according to an embodiment of the present invention;
FIG. 14 is a schematic diagram of a display interface according to an embodiment of the present invention;
FIG. 15 is a schematic diagram of a display interface according to an embodiment of the present invention;
FIG. 16 is a schematic diagram of a display interface according to an embodiment of the present invention;
FIG. 17 is a schematic block diagram of an electronic device according to an embodiment of the present invention; and
FIG. 18 is a schematic diagram of a structure of an electronic device according to an embodiment of the present invention.

### DESCRIPTION OF EMBODIMENTS

To make the technical solutions in the present invention more comprehensible, the following describes embodiments of the present invention in detail with reference to the accompanying drawings.

The terms used in the embodiments of the present invention are merely for the purpose of illustrating specific embodiments, and are not intended to limit the present invention. The terms "a", "said", and "the" of singular forms used in embodiments and the appended claims of the present invention are also intended to include plural forms, unless otherwise specified in the context clearly.

It should be understood that the term "and/or" used in this specification describes only an association relationship between associated objects and represents that three relationships may exist. For example, A and/or B may represent the following three cases: Only A exists, both A and B exist, and only B exists. In addition, the character "/" in this specification generally indicates an "or" relationship between the associated objects.

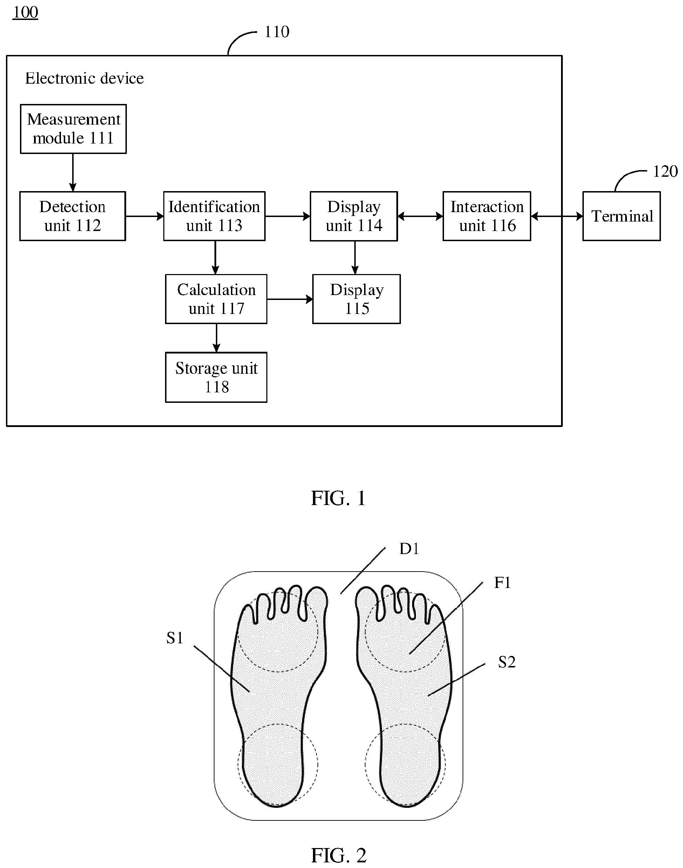

FIG. 1 is an architectural diagram of a body composition detection system according to an embodiment of the present invention. As shown in FIG. 1, the system 100 includes an electronic device 110 and a terminal 120.

In this embodiment of the present invention, the electronic device 110 includes a body fat scale. Compared with a four-electrode body fat scale and an eight-electrode body fat scale in the related technology, the body fat scale in the present invention includes a body fat scale compatible with a four-electrode mode and an eight-electrode mode.

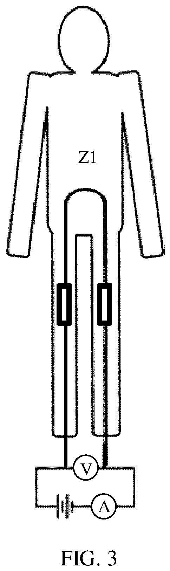

FIG. 2 is a schematic diagram of a structure of a body fat scale in a four-electrode mode, and FIG. 3 is a schematic diagram of a structure of measuring body composition of a user by the body fat scale in the four-electrode mode. As shown in FIG. 2 and FIG. 3, the body fat scale in the four-electrode mode includes a base D1 having an inductive circuit, and two standing positions S1 and S2 are disposed on the base D1. After four electrodes F1 are arranged at positions corresponding to two feet of the user, and the user stands at the corresponding standing positions S1 and S2 of the body fat scale with the two feet, a loop is formed between two legs of the user and the inductive circuit, so that a weight of and a two-leg impedance Z1 of the user can be measured, and total body composition of the user can be calculated based on the weight and the two-leg impedance Z1. The total body composition includes but is not limited to a body fat rate, a skeletal muscle mass, a protein rate, and a moisture rate. The body fat scale in the four-electrode mode does not need a handle, and therefore, it is easy to use. In addition, the electrodes are arranged at positions corresponding to the two feet of the user, so that contact surfaces between the feet and the electrodes are large. Therefore, the contact is stable, and a two-leg impedance measurement error is small. However, the body fat scale in the four-electrode mode in the related technology cannot calculate segmental body composition, and detection accuracy of the total body composition calculated by the body fat scale in the four-electrode mode is lower than that of total body composition calculated by a body fat scale in an eight-electrode mode. Therefore, in this embodiment of the present invention, the electronic device 110 is compatible with the eight-electrode mode, to resolve a problem that the body fat scale in the four-electrode mode in the related technology cannot calculate the segmental body composition.

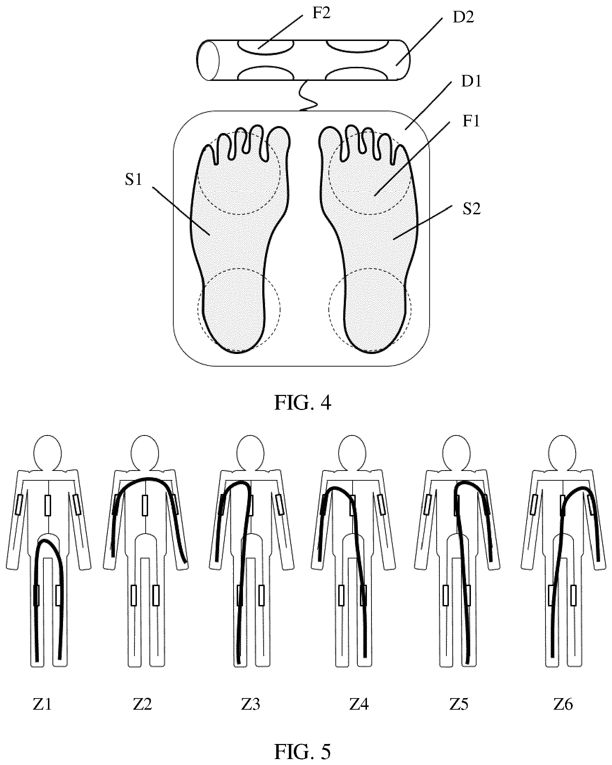

FIG. 4 is a schematic diagram of a structure of a body fat scale in an eight-electrode mode, and FIG. 5 is a schematic diagram of a structure of measuring body composition of a user by the body fat scale in the eight-electrode mode. As shown in FIG. 4 and FIG. 5, the body fat scale in the eight-electrode mode includes a base D1 and a handle D2 each having an inductive circuit, and two standing positions S1 and S2 are disposed on the base D1. After eight electrodes are arranged at positions corresponding to two feet F1 and two hands F2 of the user, and the user stands at the corresponding standing positions S1 and S2 of the body fat scale and holds the handle D1 by the hands, a loop is formed between the limbs of the user and the inductive circuits, so that a weight and segmental impedances of the user can be measured, and total body composition and segmental body composition of the user can be calculated by using the weight and the segmental impedances. The segmental impedances include a two-leg impedance Z1, a two-hand impedance Z2, a left-hand left-leg impedance Z3, a left-hand right-leg impedance Z4, a right-hand right-leg impedance Z5, and a right-hand left-hand impedance Z6. Compared with the four-electrode mode, the eight-electrode mode is more accurate in measurement, and the segmental body composition can be calculated by using the weight and the segmental impedances (Z1 to Z6). The segmental body composition may include parameters such as a left-arm fat mass, a left-arm skeletal muscle mass, a trunk fat mass, and a trunk skeletal muscle mass. Therefore, in the present invention, the body fat scale compatible with the four-electrode mode and the eight-electrode mode enables the user to select a corresponding mode to detect body composition according to a requirement.

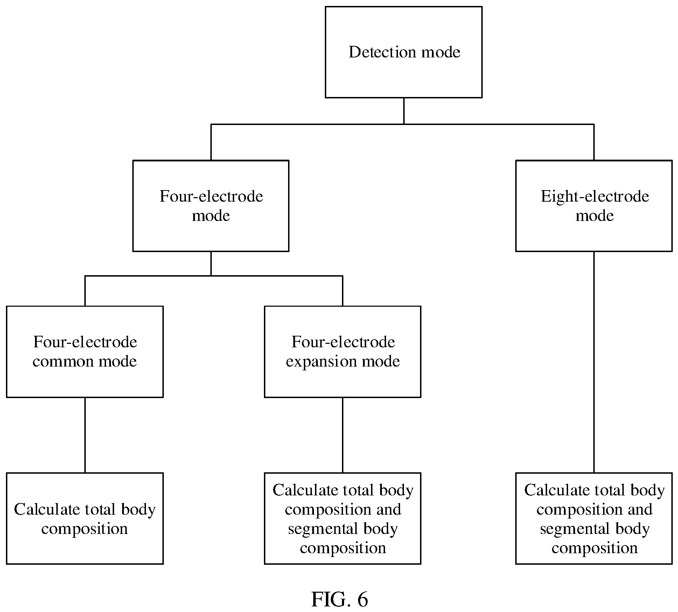

In this embodiment of the present invention, on a basis that the body fat scale is compatible with the four-electrode mode and the eight-electrode mode, the four-electrode mode is divided into a four-electrode common mode and a four-electrode expansion mode based on a principle that the body composition of the user does not change greatly in a specific time period. Therefore, when a detection mode is the four-electrode expansion mode, the total body composition and the segmental body composition can be calculated in combination with a measured weight and obtained historical segmental impedances (Z2 to Z6) in the eight-electrode mode. In this way, detection efficiency of the body composition is improved. In other words, as shown in FIG. 6, an electronic device 110 has a four-electrode mode and an eight-electrode mode, and the four-electrode mode includes a four-electrode common mode and a four-electrode expansion mode. When the detection mode includes the eight-electrode mode, total body composition and segmental body composition of a current user can be calculated. When the detection mode includes the four-electrode common mode, the total body composition of the current user can be calculated. When the detection mode includes the four-electrode expansion mode, the total body composition and the segmental body composition of the current user can be calculated. In other words, by using the eight-electrode mode, the user can accurately measure the total body composition and the segmental body composition. When the four-electrode mode is used, operations are convenient because the user does not need to use a handle, and the segmental body composition can be calculated by using the measured weight and the obtained historical segmental impedance, so that features of operation convenience and capability of calculating the segmental body composition are implemented at the same time.

The following describes detection processes in the foregoing detection modes by describing functions of units in the electronic device 110. In this embodiment of the present invention, the electronic device 110 includes a measurement module 111, a detection unit 112, an identification unit 113, a display unit 114, an interaction unit 116, a calculation unit 117, a storage unit 118, and a display 115.

In this embodiment of the present invention, the measurement unit 111 is configured to measure a weight and at least one impedance of the current user. Specifically, the measurement unit 111 includes a weight measurement module and a fat measurement module. The weight measurement module is configured to measure the weight of the current user. The fat measurement module is configured to measure the at least one impedance of the current user. The at least one impedance includes a plurality of segmental impedances or a first impedance. Specifically, when the detection mode includes the four-electrode mode, the weight and the first impedance of the current user can be measured. When the detection mode includes the eight-electrode mode, the weight and the segmental impedances of the current user can be measured.

In this embodiment of the present invention, the detection unit 112 is configured to detect a detection mode used by the current user. The detection unit may include the four-electrode mode and the eight-electrode mode. Specifically, in an optional solution, the detection unit 112 is further configured to: determine whether each segmental impedance is within a preset range; and if it is determined that each impedance is within the preset range, determine that the detection mode used by the current user is the eight-electrode mode; or if it is determined that each impedance other than the first impedance is not within the preset range, determine that the detection mode used by the current user is the four-electrode mode. In another optional solution, the detection unit 112 is alternatively configured to: detect whether the handle is lifted; and if it is detected that the handle is lifted, determine that the detection mode used by the current user is the eight-electrode mode; or if it is detected that the handle is not lifted, determine that the detection mode used by the current user is the four-electrode mode.

The identification unit 113 in this embodiment of the present invention is configured to perform identity identification on the current user to generate a user identification result, and in the process of performing identity identification on the current user to generate the user identification result, determine whether a user set can be determined, to detect whether detection behavior of the current user is correct. Specifically, the identification unit 113 is configured to perform identity identification on the current user based on the detection mode, the measured weight and at least one impedance of the current user, and obtained user data of a plurality of users, to generate the user identification result.

When the detection mode includes the eight-electrode mode, the identification unit 113 is specifically configured to: determine a first user set based on the measured weight of the current user and historical weights of the plurality of users, where the plurality of users include the current user; determine a second user set based on the first user set, the measured weight of the current user, the first impedance, and a historical first impedance, a historical body fat rate, and user attribute information of each user; determine a third user set based on the second user set, the measured weight of the current user, the plurality of segmental impedances, and the historical body fat rate and the user attribute information of each user; and perform identity identification on the current user based on the third user set, to generate the user identification result.

When the detection mode includes the four-electrode mode, the identification unit 113 is specifically configured to: determine a first user set based on the measured weight of the current user and historical weights of the plurality of users, where the plurality of users include the current user; determine a second user set based on the first user set, the measured weight of the current user, the first impedance, and a historical first impedance, a historical body fat rate, and user attribute information of each user; and perform identity identification on the current user based on the second user set, to generate the user identification result.

Based on the foregoing two detection modes, the process in which the identification unit 113 is configured to determine the first user set based on the measured weight of the current user and the historical weights of the plurality of users specifically includes: dividing an absolute value of a difference between the weight of the current user and the historical weight of each user by the historical weight of each user, to generate a first change rate corresponding to each user; and if a first change rate less than a first threshold is selected from the first change rates corresponding to the plurality of users, generating the first user set based on a user corresponding to the first change rate less than the first threshold.

The display unit 113 is configured to: if no first change rate less than the first threshold is selected from the first change rates corresponding to the plurality of users, control the display 115 to display first prompt information. The first prompt information is used to prompt a user to select/enter user attribute information, so that the calculation unit 117 subsequently calculates body composition of the user by using a measured weight, an impedance, and the user attribute information. The interaction unit 116 is configured to receive a user selection result that is entered by the current user based on the first prompt information. The user selection result may include entering a new user or selecting a user.

Based on the foregoing two detection modes, the process in which the identification unit 113 is configured to determine the second user set based on the first user set, the measured weight of the current user, the first impedance, and the historical first impedance, the historical body fat rate, and the user attribute information of each user specifically includes: calculating a first body fat rate of the current user based on the measured weight of the current user, the first impedance, and the user attribute information of each user; dividing an absolute value of a difference between the first impedance of the current user and the historical first impedance of each user by the historical first impedance of each user, to generate a second change rate corresponding to each user; dividing an absolute value of a difference between the first body fat rate of the current user and the historical body fat rate of each user by the historical body fat rate of each user, to generate a third change rate corresponding to each user; and determining the second user set based on the second change rate and the third change rate. A second change rate of a user in the first user set is less than a second threshold, and a third change rate of the user is less than a third threshold.

The display unit 114 is further configured to: if no second user set is determined based on the second change rate and the third change rate, control the display 115 to display second prompt information. The second prompt information is used to prompt a user to check whether two feet stand correctly, whether heels are in contact, and whether the two feet are wet, so that after the user eliminates these problems, the calculation module 115 re-measures the user. This ensures accuracy of a measured impedance. The interaction unit 116 is further configured to receive a user selection result that is entered by the current user based on the second prompt information. The user selection result may include confirming a corrected posture, and the like, so that the body fat scale performs re-measurement and determines the second user set.

Based on the foregoing two detection modes, the process in which the identification unit 113 is configured to determine the third user set based on the second user set, the measured weight of the current user, the plurality of segmental impedances, and the historical body fat rate and the user attribute information of each user specifically includes: calculating a second body fat rate of the current user based on the measured weight of the current user, the plurality of segmental impedances, and the user attribute information of each user; dividing an absolute value of a difference between the second body fat rate of the current user and the historical body fat rate of each user by the historical body fat rate of each user, to generate a fourth change rate corresponding to each user; and if a fourth change rate less than a fourth threshold is selected from the fourth change rates corresponding to the plurality of users, generating the third user set based on a user corresponding to the fourth change rate less than the fourth threshold.

The display unit 114 is further configured to: if no fourth change rate less than the fourth threshold is selected from the fourth change rates corresponding to the plurality of users, control the display 115 to display third prompt information. The third prompt information is used to prompt a user to check whether the handle is held tightly and whether a posture is correct, so that after the user eliminates these problems, the electronic device 110 re-measures the user. This ensures accuracy of a measured impedance. The interaction unit 116 is further configured to receive a user selection result that is entered by the current user based on the third prompt information. The user selection result may include confirming a corrected posture, and the like, so that the body fat scale performs re-measurement and determines the third user set.

The calculation unit 113 is configured to generate body composition of the current user in different detection modes based on obtained detection data in the different detection modes. Specifically, when the detection modes include the eight-electrode mode, the calculation unit 113 is further configured to calculate the total body composition and the segmental body composition of the current user based on the weight of the current user, the plurality of segmental impedances, and the obtained user attribute information of the current user. A current measurement time point is obtained. The current measurement time point, and the total body composition and the segmental body composition of the current user are stored into the storage unit 118. When the detection modes include the four-electrode mode, the calculation unit 113 is further configured to calculate the total body composition of the current user based on the weight of the current user, the first impedance, and the obtained user attribute information of the current user. A current measurement time point is obtained. The current measurement time point, and the total body composition of the current user are stored into the storage unit 118. Alternatively, when the detection modes include the four-electrode mode, the calculation unit 113 is further configured to calculate the total body composition and the segmental body composition of the current user based on the weight of the current user, the first impedance, the obtained impedance other than the first impedance of the current user, and the obtained user attribute information of the current user. A current measurement time point is obtained. The current measurement time point, and the total body composition and the segmental body composition of the current user are stored into the storage unit 118.

The display unit 114 is further configured to control the display 115 to display the total body composition of the current user and the obtained historical segmental body composition of the current user. The interaction unit 116 is further configured to send fourth prompt information to a user to prompt the user to obtain latest segmental body composition.

The display unit 114 is further configured to control the display 115 to display the total body composition and the segmental body composition of the current user. The interaction unit 116 is further configured to send fifth prompt information to the user to prompt the user to obtain latest segmental body composition.

When the detection modes include the four-electrode mode, the detection unit 112 is further configured to: obtain a current measurement time point, and determine whether a time interval between a measurement time point at which the eight-electrode mode is used for measurement last time and the current measurement time point is less than a preset time period; and if it is determined that the time interval between the measurement time point at which the eight-electrode mode is used last time and the current measurement time point is greater than the preset time period, enter a four-electrode common mode, and continue to perform the step of calculating the total body composition of the current user based on the weight of the current user, the first impedance, and the obtained user attribute information of the current user; or if it is determined that the time interval between the measurement time point at which the eight-electrode mode is used last time and the current measurement time point is less than the preset time period, enter a four-electrode expansion mode, and continue to perform the step of calculating the total body composition and the segmental body composition of the current user based on the weight of the current user, the first impedance, the obtained impedance other than the first impedance, and the obtained user attribute information of the current user.

The terminal 120 may be a handheld terminal of the current user, for example, a mobile phone terminal, a tablet computer, a notebook computer, augmented reality (augmented reality, AR for short), or virtual reality (virtual reality, VR for short). The terminal 120 may be configured to perform functions of the interaction unit 116 in the electronic device 110. In other words, when the electronic device 110 sends the first prompt information, the second prompt information, the third prompt information, the fourth prompt information, or the fifth prompt information to the user, the terminal 120 is configured to display the first prompt information, the second prompt information, the third prompt information, the fourth prompt information, or the fifth prompt information.

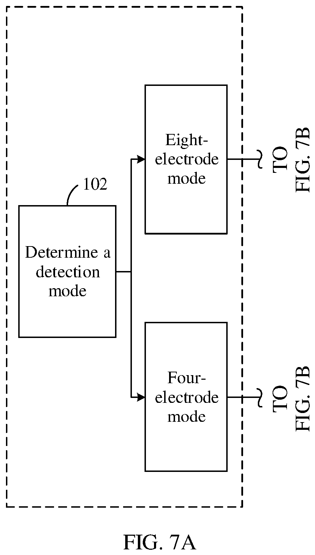

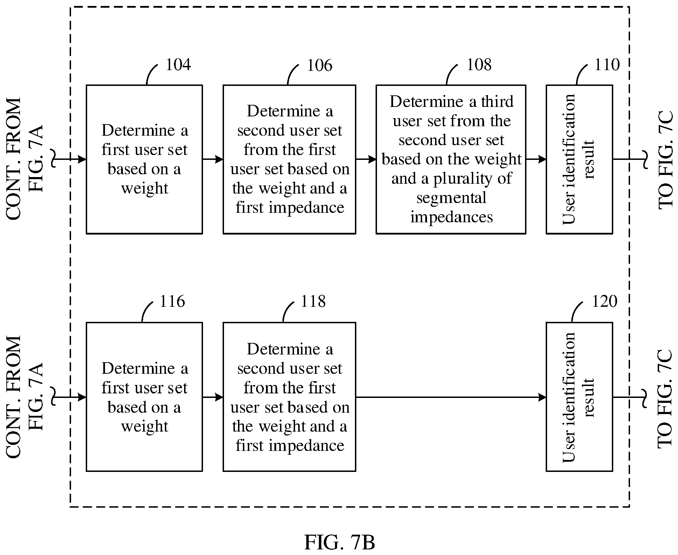

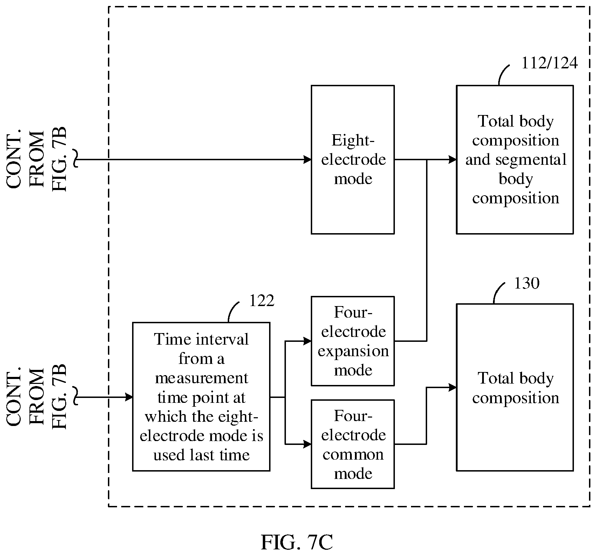

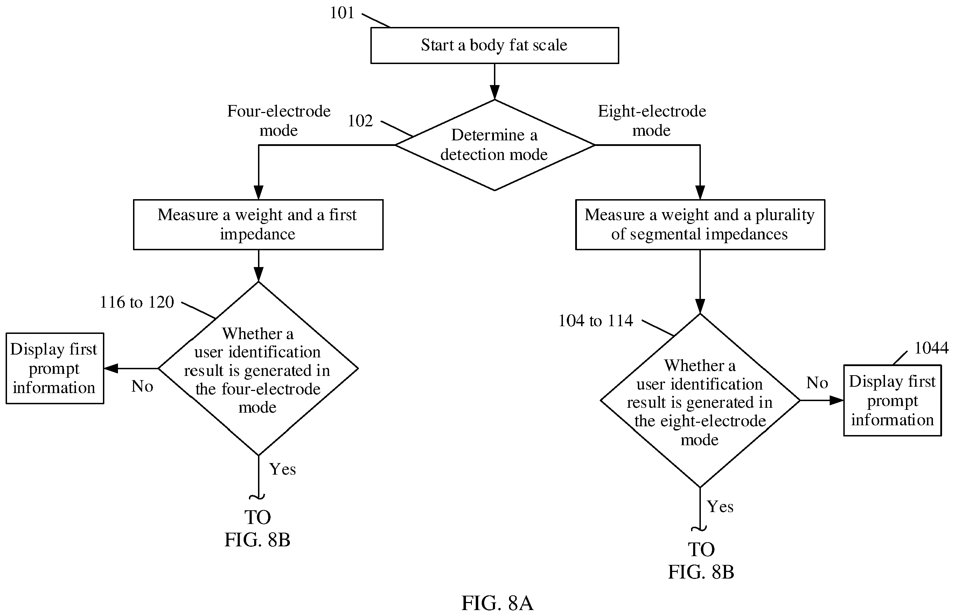

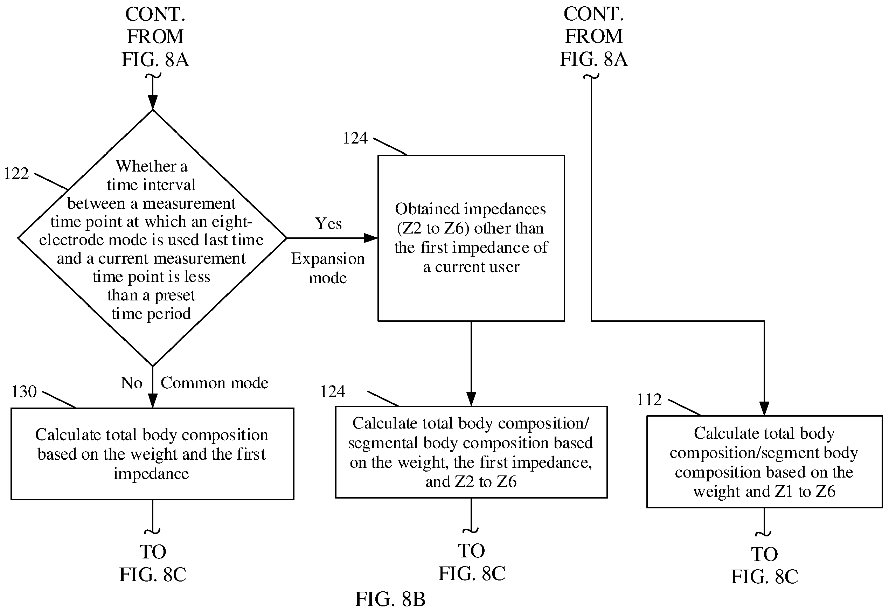

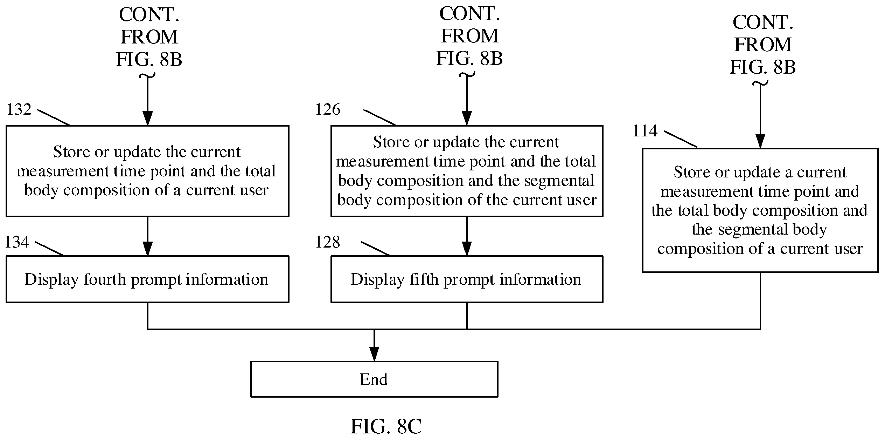

In this embodiment of the present invention, by using the foregoing system architecture 100, when user identity is identified, body composition of a current user in different detection modes is generated based on obtained detection data in the different detection modes. In this way, accuracy and efficiency of body composition detection are improved. The following describes in detail a process of a body composition detection method, including step 102 to step 134, with reference to FIG. 7A, FIG. 7B, and FIG. 7C, and FIG. 8A, FIG. 8B, and FIG. 8C.

FIG. 7A, FIG. 7B, and FIG. 7C, and FIG. 8A, FIG. 8B, and FIG. 8C are flowcharts of a body composition detection method according to an embodiment of the present invention. As shown in FIG. 7A, FIG. 7B, and FIG. 7C, and FIG. 8A, FIG. 8B, and FIG. 8C, the method includes the following steps.

Step 102: Detect that a detection mode used by a current user includes an eight-electrode mode or a four-electrode mode; and if the detection mode includes the eight-electrode mode, perform step 104; or if the detection mode includes the four-electrode mode, perform step 116.

In this embodiment of the present invention, the steps are performed by an electronic device 110, and the electronic device 110 includes a body fat scale. A four-electrode body fat scale or an eight-electrode body fat scale is usually used as a body fat scale in a related technology. However, the body fat scale in the present invention includes a body fat scale compatible with a four-electrode mode and an eight-electrode mode. Compared with a single-mode body fat scale in the related technology, the body fat scale that uses a multi-mode compatible body fat scale in the present invention has stronger practicability, and can select, according to a user requirement, a corresponding mode to detect body composition, which improves detection -side efficiency during body composition detection, and further improves user experience. It should be noted that, in addition to being compatible with the four-electrode mode and the eight-electrode mode, the body fat scale may be compatible with another mode. This is not limited in the present invention. The body fat scale compatible with the four-electrode mode and the eight-electrode mode is merely an example for description.

In this embodiment of the present invention, before step 102 is performed, the method further includes:
Step 101: Start the body fat scale, and measure a weight and at least one impedance of the current user, where a measurement unit of the body fat scale is woken up to measure the weight and at least one impedance of the current user.

In this embodiment of the present invention, for example, as shown in FIG. 2, in the four-electrode mode, when the current user stands on the body fat scale with the two legs, the weight and a first impedance of the current user can be measured. The first impedance may include the two-leg impedance Z1. As shown in FIG. 4, in the eight-electrode mode, because the handle is additionally disposed, the weight and the plurality of segmental impedances of the current user can be measured. The plurality of segmental impedances include the first impedance and an impedance other than the first impedance. For example, the plurality of segmental impedances include the two-leg impedance Z1, the two-hand impedance Z2, the left-hand left-leg impedance Z3, the left-hand right-leg impedance Z4, the right-hand right-leg impedance Z5, and the right-hand left-hand impedance Z6.

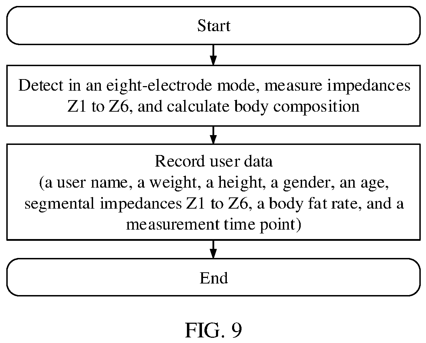

It should be noted that when a user uses the electronic device 110 to detect body composition for the first time, the electronic device 110 sends prompt information to the user, to prompt the user to use the eight-electrode mode. Specifically, a procedure of the body composition detection method is shown in FIG. 9. A weight and a plurality of segmental impedances (Z1 to Z6) of a current user are measured by using a body fat scale in an eight-electrode mode. Body composition of the current user is calculated based on the measured weight and the plurality of segmental impedances (Z1 to Z6). The body composition may include total body composition and segmental body composition. In addition, user data of the current user is stored. The user data includes a user name, a weight, a height, a gender, an age, the segmental impedances (Z1 to Z6), a body fat rate, a measurement time point, body composition, and the like, so that when the user subsequently detects the body composition by using the electronic device 110 again, identity identification can be quickly performed on the user. The total body composition and the segmental body composition of the user are quickly calculated in a four-electrode expansion mode based on the measured weight and a first impedance (Z1) of the user and with reference to information such as a historical impedance in the user data of the user, to reduce time consumed in body composition detection and improve efficiency of the body composition detection. Further, in a four-electrode common mode, the total body composition of the user is quickly calculated based on the measured weight and the first impedance (Z1) of the user, and the segmental body composition of the user is quickly obtained from the user data, to reduce time consumed in body composition detection and improve efficiency of the body composition detection.

In this embodiment of the present invention, in the process of performing step 102, the detection mode used by the current user may be detected in a plurality of manners. In an optional solution, the detection mode used by the current user may be detected by determining whether each impedance (Z1 to Z6) is within a preset range. If it is determined that each impedance (Z1 to Z6) is within the preset range, the detection mode used by the current user is determined as the eight-electrode mode. If it is determined that each impedance (Z2 to Z6) other than the first impedance is not within the preset range, the detection mode used by the current user is determined as the four-electrode mode.

In the foregoing optional solution, it should be noted that in the eight-electrode mode, the body fat scale can measure the weight and the plurality of segmental impedances (Z1 to Z6) of the current user. Therefore, if it is determined that each impedance is within the preset range, the detection mode used by the current user is determined as the eight-electrode mode. In the four-electrode mode, the body fat scale can measure only the weight and the first impedance (Z1) of the current user. In other words, in the four-electrode mode, the impedances (Z2 to Z6) other than the first impedance cannot be obtained. Therefore, it is determined that each impedance (Z2 to Z6) other than the first impedance is not within the preset range, and the detection mode used by the current user is determined as the four-electrode mode. In this optional solution, because there is a problem that impedance measurement is inaccurate due to an incorrect user operation, another optional solution may be used to detect the detection mode used by the current user.

In another optional solution, whether the handle is lifted may be detected. If it is detected that the handle is lifted, the detection mode used by the current user is determined as the eight-electrode mode; or if it is detected that the handle is not lifted, the detection mode used by the current user is determined as the four-electrode mode.

In the foregoing another optional solution, it should be noted that in the eight-electrode mode, the user needs to hold the handle and stretch the handle, so that the body fat scale can measure the weight of the current user and the plurality of segmental impedances. In the process of detecting the body composition by the body fat scale in the eight-electrode mode, the method further includes: if it is detected that a time interval in which the user does not use the handle exceeds a threshold, sending prompt information to the terminal to prompt the user to perform measurement by using the handle, to obtain an accurate total/segmental body composition. However, in the four-electrode mode, the user does not need to hold the handle. Therefore, the body fat scale can measure only the weight of the current user and the first impedance. The first impedance includes a two-leg impedance. Therefore, the detection mode used by the current user can be determined only by detecting whether the handle is lifted.

During actual application, the eight-electrode mode has a high requirement on a two-arm posture and a handle holding posture of the user, and it cannot be determined that body composition measured in the eight-electrode mode is accurate only by determining whether the handle is lifted. Therefore, identity identification needs to be performed on the current user in a subsequent step. In a process of identifying the user identity, whether the two-arm posture and the handle holding posture of the current user are correct is determined. In this way, when the two-arm posture and the handle holding posture of the current user are correct, accuracy of measured body composition can be improved.

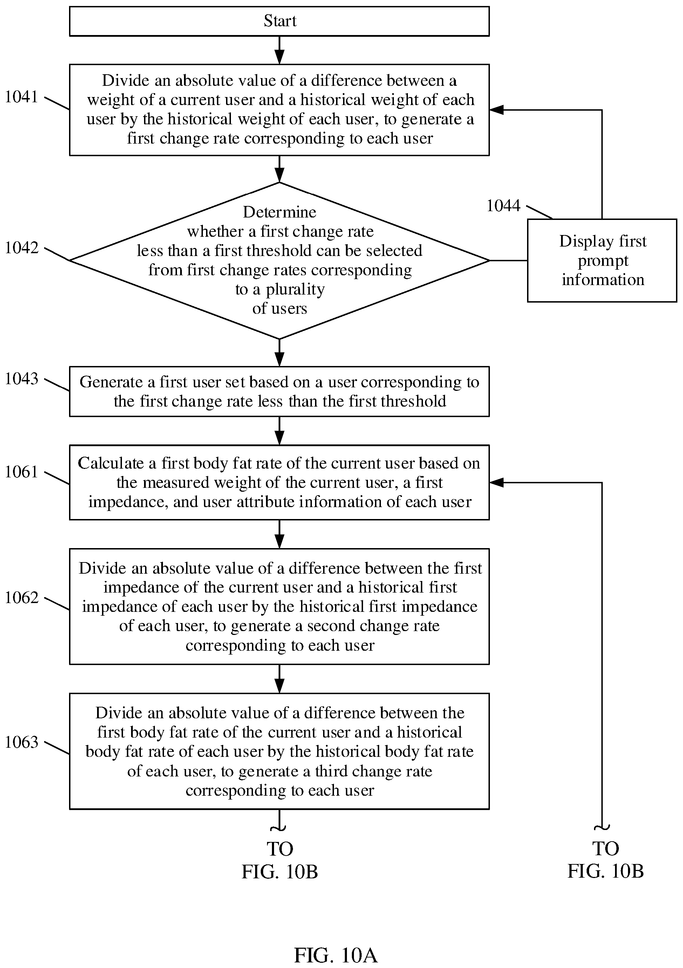

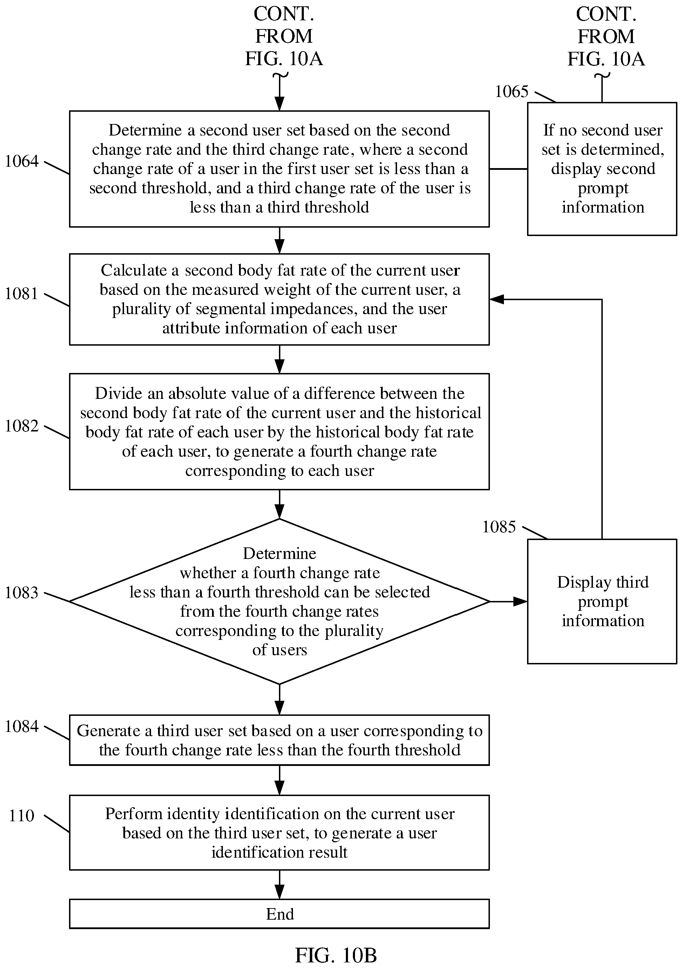

In this embodiment of the present invention, if the detection mode includes the eight-electrode mode, the following step 104 to step 110 are performed, so that identity identification can be performed on the user to generate an identity identification result. FIG. 10A and FIG. 10B are a flowchart of an identity identification method in an eight-electrode mode according to an embodiment of the present invention. As shown in FIG. 9, the method specifically includes the following step 104 to step 110.

Step 104: Determine a first user set based on the measured weight of the current user and historical weights of a plurality of users, where the plurality of users include the current user.

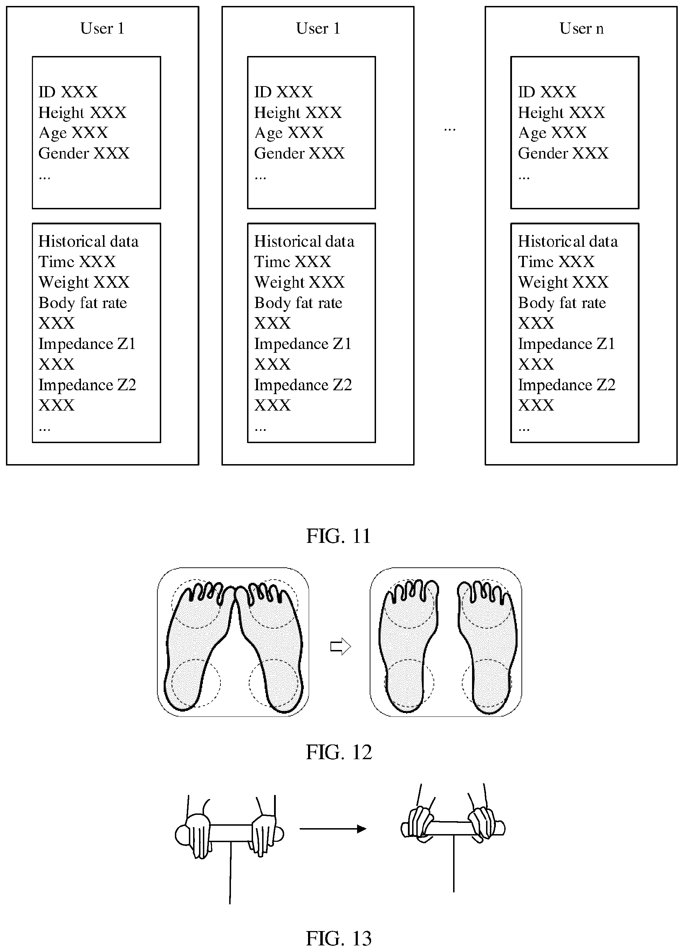

In this embodiment of the present invention, as shown in FIG. 11, the storage unit 118 stores user data of the plurality of users, and the user data of the plurality of users is obtained by using the storage unit 118. The user data includes the historical weights.

In this embodiment of the present invention, step 104 specifically includes:
Step 1041: Divide an absolute value of a difference between the weight of the current user and the historical weight of each user by the historical weight of each user, to generate a first change rate corresponding to each user.

In this embodiment of the present invention, the historical weight of each user in the user data is polled, and the absolute value of the difference between the current weight of the user and the historical weight of each user is divided by the historical weight of each user, to generate the first change rate ΔWi corresponding to each user. The first change rate is used to indicate a change rate between the weight of the current user and the historical weight of each user.

For example, the measured weight of the current user is 50 kg, and the obtained user data includes historical weights of four users. The historical weights of a user A, a user B, a user C, and a user D respectively include 40 kg, 51 kg, 60 kg, and 70 kg. The historical weight of each user is polled by using the measured weight of the current user, and a change rate of the weight of the current user relative to the user is calculated. A specific polling calculation process may include the following steps:
Step 1: Divide an absolute value of a difference between the weight 50 kg of the current user and the historical weight 40 kg of the user A by the historical weight 40 kg of the user A, to generate a first change rate ΔW1 corresponding to the user A.

The first change rate corresponding to the user A is |50 kg - 40 kg|/40 kg = 25%.

Step 2: Divide an absolute value of a difference between the current weight 50 kg of the user and the historical weight 51 kg of the user B by the historical weight 51 kg of the user B, to generate a first change rate ΔW2 corresponding to the user B.

The first change rate corresponding to the user B is |50 kg - 51 kg|/40 kg = 2.5%.

Step 3: Divide an absolute value of a difference between the current weight 50 kg of the user and the historical weight 60 kg of the user C by the historical weight 60 kg of the user C, to generate a first change rate ΔW3 corresponding to the user C.

The first change rate corresponding to the user C is |50 kg - 60 kg|/40 kg = 25%.

Step 4: Divide an absolute value of a difference between the current weight 50 kg of the user and the historical weight 70 kg of the user D by the historical weight 70 kg of the user D, to generate a first change rate ΔW4 corresponding to the user D.

The first change rate corresponding to the user D is |50 kg - 70 kg|/40 kg = 50%.

It should be noted that, if the user data further includes a historical weight of another user, polling calculation continues until the historical weight of each user in the user data is calculated in a polling manner.

Step 1042: Determine whether a first change rate less than a first threshold can be selected from the first change rates corresponding to the plurality of users; and if yes, perform step 1043; or if no, perform step 1044.

In this embodiment of the present invention, for example, the first threshold WT includes 30%. If it is determined that the first change rate less than the first threshold is selected from the first change rates corresponding to the plurality of users, it indicates that the first user set can be determined, so as to perform identity identification on the current user based on the first user set to generate a user identification result in a subsequent step. If it is determined that no first change rate less than the first threshold is selected from the first change rates corresponding to the plurality of users, it indicates that the first user set is empty, and identity identification cannot be performed on the current user. Therefore, step 1044 needs to be performed.

Step 1043: Generate the first user set based on a user corresponding to the first change rate less than the first threshold.

In this embodiment of the present invention, based on the calculation result of the foregoing steps and by performing step 1042, the first change rates corresponding to the user A, the user B, and the user C are all less than 30%, and therefore, the user A, the user B, and the user C are determined as the first user set S1. The first user set includes the current user, that is, one of the user A, the user B, and the user C is the current user. Subsequent steps need to be performed to continue to identify the current user to generate an identity identification result.

Step 1044: Display first prompt information, receive a user selection result that is entered by the current user based on the first prompt information, and continue to perform step 1041.

In this embodiment of the present invention, the display unit 114 is configured to control the display 115 to display the first prompt information. The first prompt information is used to prompt the user to select a user or enter a new user. The user selects a user or enters user attribute information, to help identify the user, and then determine user data corresponding to the user, which helps subsequently calculate body composition. Further, in addition to displaying the first prompt information on the electronic device 110, the first prompt information may be further displayed on a handheld terminal 110 of the user, and a user selection result that is entered by the current user based on the first prompt information is received. A display device is not limited in the present invention.

It should be noted that a reason why no first change rate less than the first threshold is selected from the first change rates corresponding to the plurality of users may include the following several cases. In one case, because the current user uses the electronic device 110 for the first time, the storage unit 118 of the electronic device 110 does not store the user data of the current user. Therefore, the current user needs to enter the user attribute information. The user attribute information includes parameters such as a height, a gender, and an age. In another case, because a time interval at which the current user uses the electronic device 110 to measure body composition is relatively long, the electronic device 110 does not select the first change rate less than the first threshold from the first change rates corresponding to the plurality of users. Therefore, the current user needs to manually select a user, and determine user data of the selected user as the user data of the current user, to help subsequently calculate the body composition.

Step 106: Determine a second user set based on the first user set, the measured weight of the current user, a first impedance, and a historical first impedance, a historical body fat rate, and the user attribute information of each user.

In this embodiment of the present invention, the storage unit 118 stores user data of the plurality of users, and the user data of the plurality of users is obtained by using the storage unit 118. The user data includes the historical first impedance, the historical body fat rate, and the user attribute information, and the user attribute information includes parameters such as an age, a height, and a gender.

In this embodiment of the present invention, step 106 specifically includes:
Step 1061: Calculate a first body fat rate of the current user based on the measured weight of the current user, the first impedance, and the user attribute information of each user.

In this embodiment of the present invention, the first body fat rate is used to indicate a proportion of a fat weight in a human body to a total weight of the human body. The first body fat rate is one parameter in the body composition. Specifically, the first body fat rate FRj can be calculated by using the measured weight of the current user, the first impedance, and the user attribute information of the current user.

Because no user identification result is generated when step 1061 is performed, the user attribute information of the current user cannot be determined. For example, the user attribute information includes parameters such as a height and an age. Therefore, in the process of performing step 1061, the user attribute information of each user in the user data needs to be obtained in a polling manner, and the first body fat rate FRj of the current user is calculated based on the measured weight of the current user, the first impedance, and the user attribute information of each user. For example, user attribute information of a user A, a user B, and a user C is obtained. The user attribute information of the user A includes a height of the user A and an age of the user A. The user attribute information of the user B includes a height of the user B and an age of the user B. The user attribute information of the user C includes a height of the user C and an age of the user C. Therefore, the calculating the first body fat rate of the current user may specifically include the following steps:
Step 1: Calculate a first body fat rate FR1 of the current user based on the measured weight of the current user, the first impedance, and the obtained height and age of the user A. It should be noted that the first body fat rate is not a real first body fat rate and may be understood as a temporary first body fat rate. A purpose of calculating the temporary first body fat rate is to facilitate calculation of a third change rate in a subsequent step.

Step 2: Calculate a first body fat rate FR2 of the current user based on the measured weight of the current user, the first impedance, and the obtained height and age of the user B.

Step 3: Calculate a first body fat rate FR3 of the current user based on the measured weight of the current user, the first impedance, and the obtained height and age of the user C.

It should be noted that, if the user data further includes user attribute information of another user, polling calculation continues until polling calculation on the user attribute information of each user in the user data is completed, so that a plurality of first body fat rates of the current user can be obtained. In addition, the user attribute information may further include another parameter other than the height and the age, for example, a parameter such as a gender, which is not limited in the present invention.

Step 1062: Divide an absolute value of a difference between the first impedance of the current user and the historical first impedance of each user by the historical first impedance of each user, to generate a second change rate corresponding to each user.

In this embodiment of the present invention, the historical first impedance of each user may be obtained from the user data stored in the storage unit 118. The second change rate is used to indicate a change rate between the first impedance of the current user and the historical first impedance of each user. For the process of calculating the second change rate, refer to the process of calculating the first change rate in step 1041. A difference lies only in that in step 1062, a change rate between the first impedance of the current user and the historical first impedance of each user is calculated, but in step 1041, a change rate between the weight of the current user and the historical weight of each user is calculated.

Step 1063: Divide an absolute value of a difference between the first body fat rate of the current user and the historical body fat rate of each user by the historical body fat rate of each user, to generate a third change rate corresponding to each user.

In this embodiment of the present invention, the historical body fat rate of each user may be obtained from the user data stored in the storage unit 118. Specifically, the historical body fat rate corresponding to each user is calculated based on the historical weight, the historical first impedance, and the user attribute information of each user. The third change rate is used to indicate a change rate between the first body fat rate of the current user and the historical body fat rate of each user.

For example, three first body fat rates of the current user are calculated in step 1061. For example, the three first body fat rates include a first body fat rate FR1, a first body fat rate FR2, and a first body fat rate FR3. The first body fat rate FR1 is calculated by using the weight of the current user, the first impedance, and the user attribute information of the user A. The first body fat rate FR2 is calculated by using the weight of the current user, the first impedance, and the user attribute information of the user B. The first body fat rate FR3 is calculated by using the weight of the current user, the first impedance, and the user attribute information of the user C. Therefore, when the change rate between the first body fat rate of the current user and the historical body fat rate of each user is calculated, one-to-one correspondence calculation needs to be performed. A specific calculation process may include the following steps.

Step 1: Divide an absolute value of a difference between the first body fat rate FR1 and the historical body fat rate of the user A by the historical body fat rate of the user A, to generate a third change rate ΔFR1 corresponding to the user A.

It should be noted that the first body fat rate FR1 is calculated based on the measured weight of the current user, the first impedance, and the obtained height and age of the user A. Therefore, an absolute value of a difference between the first body fat rate FR1 and the historical body fat rate of the user A needs to be divided by the historical body fat rate of the user A, to generate the third change rate ΔFR1 corresponding to the user A, so as to implement one-to-one correspondence calculation.

Step 2: Divide an absolute value of a difference between the first body fat rate FR2 and the historical body fat rate of the user B by the historical body fat rate of the user B, to generate a third change rate ΔFR2 corresponding to the user B.

Step 1: Divide an absolute value of a difference between the first body fat rate FR3 and the historical body fat rate of the user C by the historical body fat rate of the user C, to generate a third change rate ΔFR3 corresponding to the user C.

It should be noted that, if the user data further includes user attribute information of another user, the polling calculation continues until the polling calculation of the user attribute information of each user in the user data is completed, so that the third change rate corresponding to each user can be obtained.

Step 1064: Determine the second user set based on the second change rate and the third change rate, where a second change rate of a user in the first user set is less than a second threshold, and a third change rate of the user is less than a third threshold.

In this embodiment of the present invention, the second threshold and the third threshold may be set according to a requirement. This is not limited in the present invention. For example, in the second change rate and the third change rate corresponding to each user according to step 1064 and step 1065, if only the second change rate of the user B is less than the second threshold and the third change rate of the user B is less than the third threshold, the user B is determined as the second user set. If a second change rate of another user is less than the second threshold and the third change rate is less than the third threshold, the user is added to the second user set.

In this embodiment of the present invention, the method further includes:
Step 1065: If no second user set is determined based on the second change rate and the third change rate, display second prompt information, receive a user selection result that is entered by the current user based on the second prompt information, and continue to perform step 1061.

In this embodiment of the present invention, if no second user set is determined based on the second change rate and the third change rate, it indicates that no user identification result is generated. Therefore, the second prompt information needs to be displayed, so that the user enters the user selection result based on the second prompt information. The second prompt information is used to prompt the user to check whether two feet stand correctly, whether heels are in contact, and whether the two feet are wet, so that after the user eliminates these problems, the electronic device 110 re-measures the user. This ensures accuracy of a measured impedance. Further, in addition to displaying the first prompt information on the electronic device 110, the first prompt information may be further displayed on a handheld terminal 110 of the user, and a user selection result that is entered by the current user based on the first prompt information is received. A display device is not limited in the present invention.

It should be noted that a display manner of the second prompt information may include a form such as a voice, an image, or text. For example, as shown in FIG. 12, the user is prompted, in a form of an image, to separate the feet. Alternatively, the user is prompted in a form of text: Please separate the feet. In addition, a manner of combining a plurality of forms may be further included, for example, a manner of combining an image and text. In this way, the user can quickly correct a posture, to improve detection efficiency. Compared with a correct operation of notifying a user of a standing posture in advance in the related technology, in this embodiment of the present invention, the user can be prompted in a detection process, which avoids a large impedance detection error and improves body composition detection efficiency.

Step 108: Determine a third user set based on the second user set, the measured weight of the current user, the plurality of segmental impedances, and the historical body fat rate and the user attribute information of each user.

In this embodiment of the present invention, an objective of performing step 108 is to determine a more accurate third user set based on the second user set, to complete user identity identification and generate an identity identification structure.

In this embodiment of the present invention, step 108 specifically includes:
Step 1081: Calculate a second body fat rate of the current user based on the measured weight of the current user, the plurality of segmental impedances, and the user attribute information of each user.

In this embodiment of the present invention, for an execution process of step 1081, refer to step 1061. A difference lies in that the impedances used in step 1081 and step 1061 are different. In step 1061, the first body fat rate of the current user is calculated based on the weight of the current user, the first impedance, and the user attribute information of each user. In step 1081, the second body fat rate of the current user is calculated based on the measured weight of the current user, the plurality of segmental impedances, and the user attribute information of each user. Compared with the first body fat rate calculated by using the first impedance, the second body fat rate calculated by using the plurality of segmental impedances in step 1081 is more accurate.

Step 1082: Divide an absolute value of a difference between the second body fat rate of the current user and the historical body fat rate of each user by the historical body fat rate of each user, to generate a fourth change rate corresponding to each user.

In this embodiment of the present invention, the historical body fat rate of each user may be obtained from the user data stored in the storage unit 118. The fourth change rate is used to indicate a change rate between the second body fat rate of the current user and the historical body fat rate of each user. For an execution process of step 1082, refer to step 1064. A difference only lies in that step 1064 is based on the change rate between the first body fat rate of the current user and the historical body fat rate of each user, and step 1082 is based on the change rate between the second body fat rate of the current user and the historical body fat rate of each user.

Step 1083: Determine whether a fourth change rate less than a fourth threshold can be selected from the fourth change rates corresponding to the plurality of users; and if yes, perform step 1084; or if no, perform step 1084.

In this embodiment of the present invention, when the body composition is calculated in the eight-electrode mode, the user needs to hold the handle. Because a structure of the handle is difficult to design, and requirements on a two-arm posture and a handle holding posture of the user are high, when the user performs an incorrect operation, impedance measurement is inaccurate, and a measurement result error is large. The body fat scale in the related technology mainly notifies the user of a correct operation of a handle holding posture during measurement, but cannot ensure that the user performs a correct handle holding posture in use. In addition, different body fat scales require different postures. For example, some body fat scales require that there is an included angle 90° between two arms and the trunk, and some body fat scales require that there is an included angle 45° between two arms and the trunk. As a result, the body fat scale in the related technology easily causes a detection error. However, in this embodiment of the present invention, whether a user operation is correct can be determined by determining whether the fourth change rate less than the fourth threshold can be selected from the fourth change rates corresponding to the plurality of users. Specifically, if it is determined that the fourth change rate less than the fourth threshold can be selected from the fourth change rates corresponding to the plurality of users, it indicates that the operation is correct, and user identity can continue to be identified; or if it is determined that no fourth change rate less than the fourth threshold is selected from the fourth change rates corresponding to the plurality of users, it indicates that the user operation is incorrect, and step 1085 needs to be performed to prompt the user to adjust the posture.

Step 1084: Generate a third user set based on a user corresponding to the fourth change rate less than the fourth threshold.

In this embodiment of the present invention, because accuracy of the second body fat rate is higher, a more accurate user set can be determined based on the second user set by using the second body fat rate.

Step 1085: Display third prompt information, receive a user selection result that is entered by the current user based on the third prompt information, and continue to perform step 1081.

In this embodiment of the present invention, the display unit 114 is configured to control the display 115 to display the third prompt information. The third prompt information is used to prompt the user to check whether the handle is tightly held and whether a posture is correct, so that after the user eliminates these problems, the electronic device 110 re-measures the user, to ensure accuracy of at least one measured impedance. Further, in addition to displaying the first prompt information on the electronic device 110, the first prompt information may be further displayed on a handheld terminal 110 of the user, and a user selection result that is entered by the current user based on the first prompt information is received. A display device is not limited in the present invention. In this embodiment of the present invention, an impedance measurement error can be effectively avoided by actively identifying whether a user posture is correct. This improves body composition measurement accuracy and user identification accuracy, and gives full play to an advantage of a body fat scale in an eight-electrode mode.

It should be noted that a display manner of the third prompt information may include a form such as a voice, an image, or text. For example, as shown in FIG. 13, the user is prompted, in a form of an image, to hold the handle tightly. Alternatively, the user is prompted in a form of text: Please hold the handle tightly. In addition, a manner of combining a plurality of forms may be further included, for example, a manner of combining an image and text. In this way, the user can quickly correct a posture, to improve detection efficiency. Compared with a correct operation of notifying a user of a handle posture in advance in the related technology, in this embodiment of the present invention, the user can be prompted in a detection process, which avoids a large impedance detection error and improves body composition detection efficiency.

Step 110: Perform identity identification on the current user based on the third user set, to generate the user identification result.

In this embodiment of the present invention, as shown in FIG. 11, user data of a plurality of users is stored in a storage unit 118 of an electronic device 110. For facilitate user operations, based on a principle that a weight and a body fat rate of the user do not change greatly in a short time period, user data corresponding to the user can be automatically matched from the user data of the plurality of users, so that identity identification is performed on the current user to generate a user identification result.

In this embodiment of the present invention, in step 104 to step 110, identity identification is performed on the current user by matching weights, impedances, and body fat rates of the plurality of users in the user data against the weight, the impedance, and the body fat rate of the current user, to generate a user identification result. In addition to improving a user identification rate, it can be detected whether two feet of the user stand correctly, and whether a handle holding posture and a two-arm holding posture are correct, to avoid a problem that user identity is incorrectly identified because impedance measurement of an upper limb is inaccurate and a body fat rate is incorrectly measured due to the fact that the handle is not tightly grasped or the posture is incorrect, and improves measurement accuracy.

In this embodiment of the present invention, step 110 specifically includes:
Step 1101: Select, from the third user set, a user corresponding to a minimum fourth change rate.

Step 1102: Determine the user as the current user.

In this embodiment of the present invention, for example, the third user set includes a user A and a user B, and the user corresponding to the minimum fourth change rate is the user B. Therefore, the user B is determined as the current user. An objective of performing identity identification on the current user to generate the user identification result is to determine user data of the current user from user data of a plurality of users, to help subsequently calculate total body composition and segmental body composition of the user.

Step 112: Calculate total body composition and segmental body composition of the current user based on the weight of the current user, the plurality of segmental impedances, and the obtained user attribute information of the current user.

In this embodiment of the present invention, after performing identity identification on the current user to generate the user identification result in step 110, the user data of the current user can be determined from the user data of the plurality of users, and the user attribute information of the current user can be queried from the user data of the current user, where the user attribute information includes age, height, gender, and the like. That is, obtained detection data includes the weight of the current user, the plurality of segmental impedances, and the obtained user attribute information of the current user. In this case, the total body composition and the segmental body composition of the current user are calculated based on the weight of the current user, the plurality of segmental impedances, and the obtained user attribute information of the current user.

Step 114: Obtain a current measurement time point, and store the current measurement time point, and the total body composition and the segmental body composition of the current user.

In this embodiment of the present invention, the current measurement time point, and the total body composition and the segmental body composition of the current user are stored, so that the current user can quickly query for the historical total body composition and the historical segmental body composition of the current user when the body composition is detected next time.

In this embodiment of the present invention, after step 114, the method further includes:
Step 115: Display the total body composition and the segmental body composition of the current user.

In this embodiment of the present invention, the display unit 114 is configured to control the display 115 to display the total body composition and the segmental body composition of the current user, so that the user can obtain body composition information corresponding to the user. It should be noted that the total body composition and the segmental body composition of the current user may be displayed in the electronic device 110, or the total body composition and the segmental body composition of the current user may be displayed in the terminal 120. A display device is not limited in the present invention.

Step 116: Determine a first user set based on the measured weight of the current user and historical weights of a plurality of users, where the plurality of users include the current user.

In this embodiment of the present invention, for an execution process of step 116, refer to step 104.

Step 118: Determine a second user set based on the first user set, the measured weight of the current user, a first impedance, and a historical first impedance, a historical body fat rate, and user attribute information of each user.

In this embodiment of the present invention, for an execution process of step 118, refer to step 106.

Step 120: Perform identity identification on the current user based on the second user set, to generate a user identification result.

In this embodiment of the present invention, step 120 specifically includes:
Step 1201: Select, from the second user set, a user corresponding to a minimum third change rate.

Step 1202: Determine the user as the current user.

In this embodiment of the present invention, for example, the third user set includes a user A and a user B, and the user corresponding to the minimum fourth change rate is the user B. Therefore, the user B is determined as the current user. An objective of performing identity identification on the current user to generate the user identification result is to determine user data of the current user from user data of a plurality of users, to help subsequently calculate total body composition and segmental body composition of the user.

Step 122: Obtain a current measurement time point, and determine whether a time interval between a measurement time point at which an eight-electrode mode is used for detection last time and the current measurement time point is less than a preset time period; and if yes, enter a four-electrode expansion mode, and perform step 124; or if no, enter a four-electrode common unit, and perform step 130.

In this embodiment of the present invention, user data of a plurality of users is obtained by using the storage unit 118, where the user data includes a plurality of segmental impedances (Z1 to Z6), and the plurality of segmental impedances include impedances (Z2 to Z6) other than a first impedance.

Step 124: Calculate the total body composition and the segmental body composition of the current user based on the weight of the current user, the first impedance, the obtained impedance other than the first impedance of the current user, and the obtained user attribute information of the current user.

In this embodiment of the present invention, when the detection mode is in the four-electrode expansion mode, the segmental impedance (Z2 to Z6) measured in the eight-electrode mode, the measured weight of the current user, the first impedance, and the obtained user attribute information of the current user may be obtained, so that the total body composition and the segmental body composition can be calculated. This improves body composition detection efficiency.

Step 126: Store the current measurement time point, and the total body composition and the segmental body composition of the current user.

Step 128: Display the total body composition and the segmental body composition of the current user, and send fifth prompt information to the user, to prompt the user to obtain latest segmental body composition.

In this embodiment of the present invention, the display unit 114 is configured to control the display 115 to display the total body composition and the segmental body composition of the current user. Further, in addition to displaying the total body composition and the segmental body composition of the current user by the electronic device 110, the total body composition and the segmental body composition of the current user may be alternatively displayed in the handheld terminal 110 of the user. A display device is not limited in the present invention.

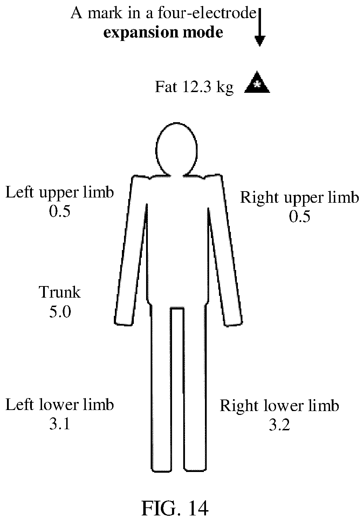

In this embodiment of the present invention, the fifth prompt information is used to prompt the user to obtain the latest segmental body composition. In a four-electrode expansion mode, the total body composition and the segmental body composition of the current user are calculated by using the measured weight of the current user, the first impedance, and the obtained segmental impedances (Z2 to Z6) measured in the eight-electrode mode, so that the calculated segmental body composition is not real segmental body composition of the current user. Therefore, the fifth prompt information may be displayed on the display 115 of the electronic device 110, to prompt the user to obtain the latest segmental body composition. A specific display manner may include: when the total body composition and the segmental body composition of the current user are displayed, adding a mark to describe the segmental body composition, for example, as shown in FIG. 14, adding * to an interface to indicate that the segmental body composition is calculated with reference to the obtained historical impedance data (Z2 to Z6); and prompting the user to use the handle to obtain the latest segmental body composition.

Step 130: Calculate the total body composition of the current user based on the weight of the current user, the first impedance, and the obtained user attribute information of the current user.

Step 132: Store the current measurement time point and the total body composition of the current user.

Step 134: Display the total body composition of the current user and the obtained historical segmental body composition of the current user, and send fourth prompt information to the user to prompt the user to obtain the latest segmental body composition.

In this embodiment of the present invention, the display unit 114 is configured to control the display 115 to display the total body composition and the segmental body composition of the current user. Further, in addition to displaying the total body composition and the segmental body composition of the current user by the electronic device 110, the total body composition and the segmental body composition of the current user may be alternatively displayed in the handheld terminal 110 of the user. A display device is not limited in the present invention.

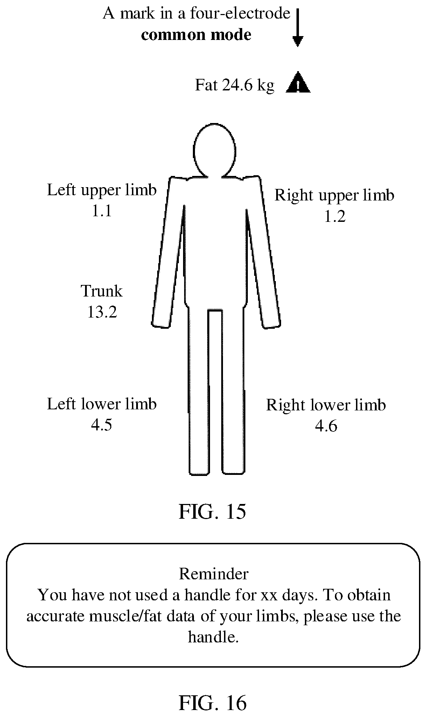

In this embodiment of the present invention, the fourth prompt information is used to prompt the user to obtain the latest segmental body composition. In the four-electrode common mode, the historical segmental body composition is obtained by using the user data, and the segmental body composition is not real segmental body composition of the current user. Therefore, the fourth prompt information may be displayed on the display 115 of the electronic device 110, to prompt the user to obtain the latest segmental body composition. A specific display manner may include: when the total body composition and the segmental body composition of the current user are displayed, adding a mark to describe the segmental body composition, for example, as shown in FIG. 15, adding ! to an interface to indicate that the segmental body composition is historical segmental body composition; and prompting the user to use the handle to obtain the latest segmental body composition.

In this embodiment of the present invention, further, the method further includes: if it is detected that the current user does not perform measurement by using the eight-electrode mode within a preset time interval, displaying sixth prompt information to prompt the user to obtain latest segmental body composition. For example, as shown in FIG. 16, in an interface, in a form of text, the user is actively prompted to use a handle to perform measurement in order to obtain accurate segmental body composition data.

In this embodiment of the present invention, in an eight-electrode detection mode, identity identification is performed on the current user by using the measured weight and at least one impedance of the current user and the obtained user data of the plurality of users, to generate the user identification result. In this way, the user identity identification accuracy is improved, and it can be detected whether the two feet of the user stand correctly, and whether the handle holding posture and the two-arm holding posture are correct, to improve body composition detection accuracy. In a four-electrode expansion detection mode, total body composition and segmental body composition are calculated by combining historical segmental impedances, so that the user obtains comprehensive data in a relatively comfortable four-electrode detection mode, to improve body composition detection efficiency.

In this embodiment of the present invention, the detection mode used by the current user is detected, identity identification is performed on the current user based on the detection mode and the measured weight and at least one impedance of the current user to generate the user identification result, and body composition of the current user in different detection modes are generated based on obtained detection data in the different detection modes. In this way, accuracy and efficiency of body composition detection are improved.

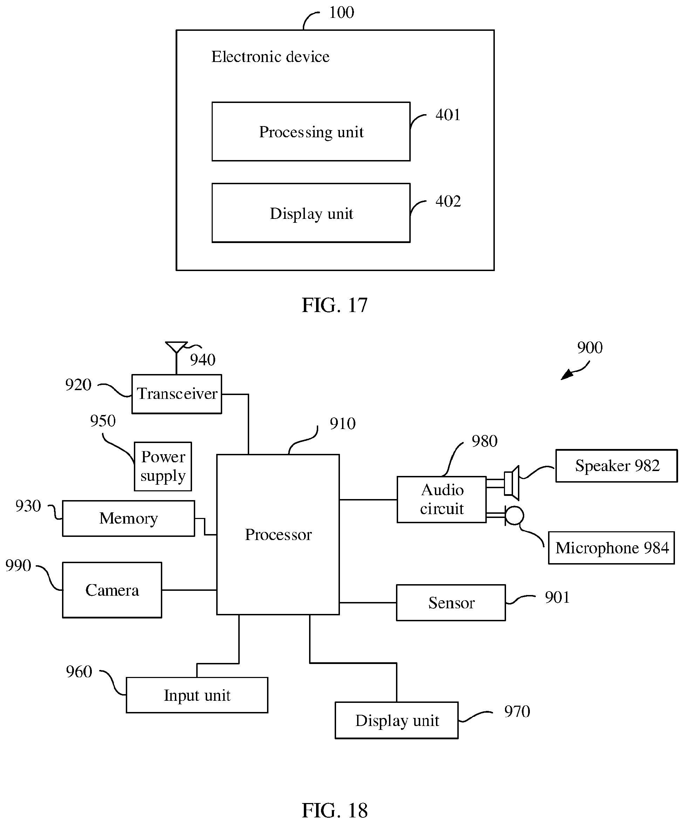

FIG. 17 is a schematic block diagram of an electronic device 110 according to an embodiment of the present invention. It should be understood that the electronic device 110 can perform the steps in the body composition detection methods in FIG. 7A, FIG. 7B, and FIG. 7C, and FIG. 8A, FIG. 8B, and FIG. 8C. To avoid repetition, details are not described herein again. As shown in FIG. 17, the electronic device 110 includes a processing unit 401 and a display unit 402.

The processing unit 401 is configured to detect a detection mode used by a current user.

The processing unit 401 is further configured to perform identity identification on the current user based on the detection mode, a measured weight and at least one impedance of the current user, and obtained user data of a plurality of users, to generate a user identification result.

The processing unit 401 is further configured to generate body composition of the current user in different detection modes based on obtained detection data in the different detection modes.

In a non-claimed possible implementation, the at least one impedance includes a plurality of segmental impedances, the plurality of segmental impedances include a first impedance, and the user data includes a historical weight, a historical first impedance, a historical body fat rate, and user attribute information. The processing unit 401 is further configured to determine a first user set based on the measured weight of the current user and the historical weights of the plurality of users who include the current user.

The processing unit 401 is further configured to determine a second user set based on the first user set, the measured weight of the current user, the first impedance, and the historical first impedance, the historical body fat rate, and the user attribute information of each user.

The processing unit 401 is further configured to determine a third user set based on the second user set, the measured weight of the current user, the plurality of segmental impedances, and the historical body fat rate and the user attribute information of each user.

The processing unit 401 is further configured to perform identity identification on the current user based on the third user set, to generate the user identification result.

In the present invention, when the detection mode includes a four-electrode mode, the at least one impedance includes a first impedance, and the user data includes a historical weight, a historical first impedance, a historical body fat rate, and user attribute information. The processing unit 401 is further configured to determine a first user set based on the measured weight of the current user and the historical weights of the plurality of users who include the current user.

The processing unit 401 is further configured to determine a second user set based on the first user set, the measured weight of the current user, the first impedance, and the historical first impedance, the historical body fat rate, and the user attribute information of each user.

The processing unit 401 is further configured to perform identity identification on the current user based on the second user set, to generate the user identification result.

In a possible implementation, the processing unit 401 is further configured to: divide an absolute value of a difference between the weight of the current user and the historical weight of each user by the historical weight of each user, to generate a first change rate corresponding to each user; and if a first change rate less than a first threshold is selected from the first change rates corresponding to the plurality of users, generate the first user set based on a user corresponding to the first change rate less than the first threshold.

In a possible implementation, the display unit 402 is configured to: if no first change rate less than the first threshold is selected from the first change rates corresponding to the plurality of users, display first prompt information; and receive a user selection result that is entered by the current user based on the first prompt information.

In a possible implementation, the processing unit 401 is further configured to: calculate a first body fat rate of the current user based on the measured weight of the current user, the first impedance, and the user attribute information of each user; divide an absolute value of a difference between the first impedance of the current user and the historical first impedance of each user by the historical first impedance of each user, to generate a second change rate corresponding to each user; divide an absolute value of a difference between the first body fat rate of the current user and the historical body fat rate of each user by the historical body fat rate of each user, to generate a third change rate corresponding to each user; and determine the second user set based on the second change rate and the third change rate. A second change rate of a user in the first user set is less than a second threshold, and a third change rate of the user is less than a third threshold.

In a possible implementation, the display unit 402 is further configured to: if no second user set is determined based on the second change rate and the third change rate, display second prompt information; and receive a user selection result that is entered by the current user based on the second prompt information.

In a possible implementation, the processing unit 401 is further configured to: calculate a second body fat rate of the current user based on the measured weight of the current user, the plurality of segmental impedances, and the user attribute information of each user; divide an absolute value of a difference between the second body fat rate of the current user and the historical body fat rate of each user by the historical body fat rate of each user, to generate a fourth change rate corresponding to each user; and if a fourth change rate less than a fourth threshold is selected from the fourth change rates corresponding to the plurality of users, generate the third user set based on a user corresponding to the fourth change rate less than the fourth threshold.

In a possible implementation, the display unit 402 is further configured to: if no fourth change rate less than the fourth threshold is selected from the fourth change rates corresponding to the plurality of users, display third prompt information; and receive a user selection result that is entered by the current user based on the third prompt information.

In a possible implementation, the processing unit 401 is further configured to select, from the third user set, a user corresponding to a minimum fourth change rate, and determine the user corresponding to the minimum fourth change rate as the current user.

In a possible implementation, the processing unit 401 is further configured to select, from the third user set, a user corresponding to a minimum fourth change rate, and determine the user corresponding to the minimum fourth change rate as the current user.

In a possible implementation, the detection mode includes an eight-electrode mode, and the detection data includes the weight of the current user, a plurality of segmental impedances, and obtained user attribute information of the current user. The processing unit 401 is further configured to calculate total body composition and segmental body composition of the current user based on the weight of the current user, the plurality of segmental impedances, and the obtained user attribute information of the current user.

In a possible implementation, the processing unit 401 is further configured to: obtain a current measurement time point, and store the current measurement time point, and the total body composition and the segmental body composition of the current user.

In a possible implementation, the detection mode includes the four-electrode mode, and the detection data includes the weight of the current user, the first impedance, and the obtained user attribute information of the current user. The processing unit 401 is further configured to calculate total body composition of the current user based on the weight of the current user, the first impedance, and the obtained user attribute information of the current user.

In a possible implementation, the detection mode includes a four-electrode mode. The processing unit 401 is further configured to: obtain a current measurement time point, and store the current measurement time point and the total body composition of the current user.

The display unit 402 is further configured to display the total body composition of the current user and obtained historical segmental body composition of the current user, and send fourth prompt information to the user to prompt the user to obtain latest segmental body composition.

In a possible implementation, the detection mode includes the four-electrode mode, and the detection data includes the weight of the current user, the first impedance, an obtained impedance other than the first impedance of the current user, and the obtained user attribute information of the current user. The processing unit 401 is further configured to calculate total body composition and segmental body composition of the current user based on the weight of the current user, the first impedance, the obtained impedance other than the first impedance of the current user, and the obtained user attribute information of the current user.

In a possible implementation, the detection mode includes the four-electrode mode. The processing unit 401 is further configured to: obtain a current measurement time point, and store the current measurement time point, and the total body composition and the segmental body composition of the current user.

The display unit 402 is further configured to display the total body composition and the segmental body composition of the current user, and send fifth prompt information to the user to prompt the user to obtain latest segmental body composition.

When the detection mode includes the four-electrode mode, the processing unit 401 is further configured to: obtain a current measurement time point, and determine whether a time interval between a measurement time point at which an eight-electrode mode is used for detection last time and the current measurement time point is less than a preset time period; and if it is determined that the time interval between the measurement time point at which the eight-electrode mode is used last time and the current measurement time point is greater than the preset time period, enter a four-electrode common mode, and continue to perform the step of calculating total body composition of the current user based on the weight of the current user, the first impedance, and the obtained user attribute information of the current user; or if it is determined that the time interval between the measurement time point at which the eight-electrode mode is used last time and the current measurement time point is less than the preset time period, enter a four-electrode expansion mode, and continue to perform the step of calculating total body composition and segmental body composition of the current user based on the weight of the current user, the first impedance, the obtained impedance other than the first impedance of the current user, and the obtained user attribute information of the current user.

In a possible implementation, the plurality of segmental impedances include an impedance other than a first impedance. The processing unit 401 is further configured to: determine whether each segmental impedance is within a preset range; and if it is determined that each impedance is within the preset range, determine that the detection mode used by the current user is an eight-electrode mode; or if it is determined that the impedance other than the first impedance is not within the preset range, determine that the detection mode used by the current user is a four-electrode mode.

In a possible implementation, for the detecting a detection mode used by a current user, the processing unit 401 is further configured to: if it is detected that a handle is lifted, determine that the detection mode used by the current user is an eight-electrode mode; or if it is detected that a handle is not lifted, determine that the detection mode used by the current user is a four-electrode mode.

In a possible implementation, the first impedance includes a two-leg impedance.

In a possible implementation, the plurality of segmental impedances further include a two-hand impedance, a left-hand left-leg impedance, a left-hand right-leg impedance, a right-hand right-leg impedance, and a right-hand left-hand impedance.

It should be understood that the electronic device 110 herein is embodied in a form of a functional unit. The term "unit" herein may be implemented in a form of software and/or hardware. This is not specifically limited. For example, the "unit" may be a software program, a hardware circuit, or a combination thereof for implementing the foregoing function. The hardware circuit may include an application-specific integrated circuit (application-specific integrated circuit, ASIC), an electronic circuit, a processor (for example, a shared processor, a dedicated processor, or a group processor) configured to execute one or more software or firmware programs and a memory, a merged logic circuit, and/or another appropriate component that supports the described function.

Therefore, the units in the examples described in embodiments of the present invention can be implemented by using electronic hardware, or a combination of computer software and electronic hardware. Whether functions are performed in a hardware or software manner depends on particular applications and design constraint conditions of the technical solutions. A person skilled in the art may use different methods to implement the described functions for each particular application.

An embodiment of the present invention further provides an electronic device. The electronic device may be a terminal device, or may be a circuit device built into the terminal device. The device may be configured to perform the functions/steps in the foregoing method embodiments.

FIG. 18 is a schematic diagram of a structure of another electronic device according to an embodiment of the present invention. As shown in FIG. 18, the electronic device 900 includes a processor 910 and a transceiver 920. Optionally, the electronic device 900 may further include a memory 930. The processor 910, the transceiver 920, and the memory 930 may communicate with each other through an internal connection path to transfer a control signal and/or a data signal. The memory 930 is configured to store a computer program. The processor 910 is configured to: invoke the computer program from the memory 930 and run the computer program.

Optionally, the electronic device 900 may further include an antenna 940, configured to send a wireless signal output by the transceiver 920.

The processor 910 and the memory 930 may be integrated into one processing apparatus, or more commonly be components independent of each other. The processor 910 is configured to execute program code stored in the memory 930 to implement the foregoing functions. During specific implementation, the memory 930 may also be integrated into the processor 910, or may be independent of the processor 910. The processor 910 may correspond to the processing unit 401 in the electronic device 110 in FIG. 17.

In addition, the electronic device 900 may further include one or more of an input unit 960, a display unit 970, an audio circuit 980, a camera 990, a sensor 901, and the like, to improve the functions of the electronic device 900. The audio circuit may further include a speaker 982, a microphone 984, and the like. The display unit 970 may include a display, and the display unit 970 may correspond to the display unit 402 in the electronic device 110 in FIG. 17.

Optionally, the electronic device 900 may further include a power supply 950, configured to supply power to various components or circuits in the terminal device.

It should be understood that the electronic device 900 shown in FIG. 18 can implement the processes of the method embodiments shown in FIG. 7A, FIG. 7B, and FIG. 7C, FIG. 8A, FIG. 8B, and FIG. 8C, and FIG. 10A and FIG. 10B. Operations and/or functions of the units in the electronic device 900 are separately intended to implement a corresponding procedure in the foregoing method embodiments. For details, refer to the descriptions in the foregoing method embodiments. To avoid repetition, detailed descriptions are properly omitted herein.

It should be understood that the processor 910 in the electronic device 900 shown in FIG. 18 may be a system on a chip (system on a chip, SOC). The processor 910 may include a central processing unit (central processing unit, CPU), and may further include another type of processor. The CPU may be referred to as a host CPU (Host CPU). A neural-network processing unit NPU 30 is mounted to the host CPU as a coprocessor, and the host CPU assigns a task. The processors work together to implement the foregoing method procedure, and each processor may selectively execute a part of a software driver.

In conclusion, some processors or processing units in the processor 910 may cooperate to implement the foregoing method procedure, and software programs corresponding to the processors or processing units may be stored in the memory 930.

The present invention further provides a computer-readable storage medium. The computer-readable storage medium stores instructions. When the instruction is run on a computer, the computer is enabled to perform the steps in the body composition detection methods shown in FIG. 7A, FIG. 7B, and FIG. 7C, FIG. 8A, FIG. 8B, and FIG. 8C, and FIG. 10A and FIG. 10B.

In the foregoing embodiments, the processor 910 may include, for example, a central processing unit (central processing unit, CPU), a microprocessor, a microcontroller, or a digital signal processor, and may further include a GPU, an NPU, and an ISP. The processor may further include a necessary hardware accelerator or a logic processing hardware circuit, for example, an application-specific integrated circuit (application-specific integrated circuit, ASIC), or one or more integrated circuits configured to control execution of programs in the technical solutions in the present invention. In addition, the processor may have a function of operating one or more software programs, and the software program may be stored in the memory.

The memory may be a read-only memory (read-only memory, ROM), another type of static storage device that can store static information and instructions, or a random access memory (random access memory, RAM) or another type of dynamic storage device that can store information and instructions, or may be an electrically erasable programmable read-only memory (electrically erasable programmable read-only memory, EEPROM), a compact disc read-only memory (compact disc read-only memory, CD-ROM) or another compact disc storage medium, an optical disc storage medium (including a compact optical disc, a laser disc, an optical disc, a digital versatile optical disc, a Blu-ray disc, and the like), a magnetic disk storage medium or another magnetic storage device, any other medium that can be used to carry or store expected program code in a form of instructions or a data structure and that can be accessed by a computer, or the like.

In embodiments of the present invention, "at least one" means one or more, and "a plurality of" means two or more. The term "and/or" describes an association relationship for describing associated objects and indicates that three relationships may exist. For example, A and/or B may indicate the following three cases: Only A exists, both A and B exist, and only B exists. A and B may be in a singular form or a plural form. The character "/" usually indicates an "or" relationship between the associated objects. At least one of the following items and similar expressions refer to any combination of the items, including a single item or any combination of plural items. For example, at least one of a, b, and c may indicate a, b, c, a and b, a and c, b and c, or a, b, and c, where a, b, and c may be singular or plural.

A person of ordinary skill in the art may be aware that, with reference to the examples described in embodiments disclosed in this specification, units and algorithm steps may be implemented by electronic hardware or a combination of computer software and electronic hardware. Whether functions are performed in a hardware or software manner depends on particular applications and design constraint conditions of the technical solutions. A person skilled in the art may use different methods to implement the described functions for each particular application.

It may be clearly understood by a person skilled in the art that, for the purpose of convenient and brief description, for a detailed working process of the foregoing system, apparatus, and unit, refer to a corresponding process in the foregoing method embodiments. Details are not described herein again.

In embodiments of the present invention, when any of the functions are implemented in a form of a software functional unit and sold or used as an independent product, the functions may be stored in a computer-readable storage medium. Based on such an understanding, the technical solutions of the present invention essentially, or the part contributing to the conventional technology, or some of the technical solutions may be implemented in a form of a software product. The computer software product is stored in a storage medium, and includes several instructions for instructing a computer device (which may be a personal computer, a server, or a network device) to perform all or some of the steps of the methods described in the embodiments of the present invention. The foregoing storage medium includes any medium that can store program code, such as a USB flash drive, a removable hard disk, a read-only memory (read-only memory, ROM), a random access memory (random access memory, RAM), a magnetic disk, or an optical disc.

The above descriptions are merely specific implementations of the present invention. The protection scope of the present invention shall be subject to the protection scope of the claims.

## Claims

1. A body composition detection method, comprising:
detecting (102) a detection mode used by a current user;
performing (110, 120) identity identification on the current user based on the detection mode, a measured weight and at least one impedance of the current user, and obtained user data of a plurality of users, to generate a user identification result; and
generating body composition of the current user in different detection modes based on obtained detection data in the different detection modes,
wherein the detection mode comprises a four-electrode mode, the at least one impedance comprises a first impedance, and the user data comprises a historical weight, a historical first impedance, a historical body fat rate, and user attribute information; and
the performing identity identification on the current user based on the detection mode, a measured weight and at least one impedance of the current user, and obtained user data of a plurality of users, to generate a user identification result comprises:
determining a first user set based on the measured weight of the current user and the historical weights of the plurality of users who comprise the current user;
determining a second user set based on the first user set, the measured weight of the current user, the first impedance, and the historical first impedance, the historical body fat rate, and the user attribute information of each user; and
performing identity identification on the current user based on the second user set, to generate the user identification result;
wherein the performing identity identification on the current user based on the second user set, to generate the user identification result comprises:
selecting, from the second user set, a user corresponding to a minimum third change rate, and determining the user corresponding to the minimum third change rate as the current user;
wherein
after the selecting, from the second user set, a user corresponding to a minimum third change rate, and determining the user corresponding to the minimum third change rate as the current user, the method further comprises:
obtaining a current measurement time point, and determining whether a time interval between a measurement time point at which an eight-electrode mode is used for detection last time and the current measurement time point is less than a preset time period; and
if it is determined that the time interval between the measurement time point at which the eight-electrode mode is used last time and the current measurement time point is greater than the preset time period, entering a four-electrode common mode, and continuing to perform the step of calculating total body composition of the current user based on the weight of the current user, the first impedance, and the obtained user attribute information of the current user; or
if it is determined that the time interval between the measurement time point at which the eight-electrode mode is used last time and the current measurement time point is less than the preset time period, entering a four-electrode expansion mode, and continuing to perform the step of calculating total body composition and segmental body composition of the current user based on the weight of the current user, the first impedance, an obtained impedance other than the first impedance of the current user, and the obtained user attribute information of the current user.

2. A body composition detection device, comprising means for:
detecting a detection mode used by a current user;
performing identity identification on the current user based on the detection mode, a measured weight and at least one impedance of the current user, and obtained user data of a plurality of users, to generate a user identification result; and
generating body composition of the current user in different detection modes based on obtained detection data in the different detection modes;
wherein the detection mode comprises a four-electrode mode, the at least one impedance comprises a first impedance, and the user data comprises a historical weight, a historical first impedance, a historical body fat rate, and user attribute information; and
the performing identity identification on the current user based on the detection mode, a measured weight and at least one impedance of the current user, and obtained user data of a plurality of users, to generate a user identification result comprises:
determining a first user set based on the measured weight of the current user and the historical weights of the plurality of users who comprise the current user;
determining a second user set based on the first user set, the measured weight of the current user, the first impedance, and the historical first impedance, the historical body fat rate, and the user attribute information of each user; and
performing identity identification on the current user based on the second user set, to generate the user identification result;
wherein the performing identity identification on the current user based on the second user set, to generate the user identification result comprises:
selecting, from the second user set, a user corresponding to a minimum third change rate, and determining the user corresponding to the minimum third change rate as the current user;
wherein
after the selecting, from the second user set, a user corresponding to a minimum third change rate, and determining the user corresponding to the minimum third change rate as the current user, the device further comprises:
obtaining a current measurement time point, and determining whether a time interval between a measurement time point at which an eight-electrode mode is used for detection last time and the current measurement time point is less than a preset time period; and
if it is determined that the time interval between the measurement time point at which the eight-electrode mode is used last time and the current measurement time point is greater than the preset time period, entering the four-electrode common mode, and continuing to perform the step of calculating total body composition of the current user based on the weight of the current user, the first impedance, and the obtained user attribute information of the current user; or
if it is determined that the time interval between the measurement time point at which the eight-electrode mode is used last time and the current measurement time point is less than the preset time period, entering a four-electrode expansion mode, and continuing to perform the step of calculating total body composition and segmental body composition of the current user based on the weight of the current user, the first impedance, an obtained impedance
other than the first impedance of the current user, and the obtained user attribute information of the current user.

## Patentansprüche

1. Körperzusammensetzung-Detektionsverfahren, umfassend:
Detektieren (102) eines durch einen gegenwärtigen Benutzer verwendeten Detektionsmodus;
Durchführen (110, 120) einer Identitätsidentifizierung an dem gegenwärtigen Benutzer basierend auf dem Detektionsmodus, einem gemessenen Gewicht und mindestens einer Impedanz des gegenwärtigen Benutzers und erlangten Benutzerdaten einer Vielzahl von Benutzern, um ein Benutzeridentifizierungsergebnis zu erzeugen; und
Erzeugen einer Körperzusammensetzung des gegenwärtigen Benutzers in verschiedenen Detektionsmodi basierend auf erlangten Detektionsdaten in den verschiedenen Detektionsmodi,
wobei der Detektionsmodus einen Vier-Elektroden-Modus umfasst, die mindestens eine Impedanz eine erste Impedanz umfasst und die Benutzerdaten ein historisches Gewicht, eine historische erste Impedanz, eine historische Körperfettrate und Benutzerattributinformationen umfassen; und
das Durchführen einer Identitätsidentifizierung an dem gegenwärtigen Benutzer basierend auf dem Detektionsmodus, einem gemessenen Gewicht und mindestens einer Impedanz des gegenwärtigen Benutzers und erlangten Benutzerdaten einer Vielzahl von Benutzern, um ein Benutzeridentifizierungsergebnis zu erzeugen, umfasst:
Bestimmen eines ersten Benutzersatzes basierend auf dem gemessenen Gewicht des gegenwärtigen Benutzers und den historischen Gewichten der Vielzahl von Benutzern, die den gegenwärtigen Benutzer umfassen;
Bestimmen eines zweiten Benutzersatzes basierend auf dem ersten Benutzersatz, dem gemessenen Gewicht des gegenwärtigen Benutzers, der ersten Impedanz und der historischen ersten Impedanz, der historischen Körperfettrate und den Benutzerattributinformationen jedes Benutzers; und
Durchführen einer Identitätsidentifizierung an dem gegenwärtigen Benutzer basierend auf dem zweiten Benutzersatz, um das Benutzeridentifizierungsergebnis zu erzeugen; wobei das Durchführen einer Identitätsidentifizierung an dem gegenwärtigen Benutzer basierend auf dem zweiten Benutzersatz, um das Benutzeridentifizierungsergebnis zu erzeugen umfasst:
Auswählen, aus dem zweiten Benutzersatz, eines Benutzers korrespondierend mit einer minimalen dritten Änderungsrate und Bestimmen des Benutzers korrespondierend mit der minimalen dritten Änderungsrate als den gegenwärtigen Benutzer;
wobei
nach dem Auswählen, aus dem zweiten Benutzersatz, eines Benutzers korrespondierend mit einer minimalen dritten Änderungsrate und Bestimmen des Benutzers korrespondierend mit der minimalen dritten Änderungsrate als den gegenwärtigen Benutzer das Verfahren ferner umfasst:
Erlangen eines gegenwärtigen Messungszeitpunkts und Bestimmen, ob ein Zeitintervall zwischen einem Messungszeitpunkt, zu dem ein Acht-Elektroden-Modus zur Detektion zuletzt verwendet wurde, und dem gegenwärtigen Messungszeitpunkt kleiner als eine im Voraus eingestellte Zeitperiode ist; und
wenn bestimmt wird, dass das Zeitintervall zwischen dem Messungszeitpunkt, zu dem der Acht-Elektroden-Modus zuletzt verwendet wurde, und dem gegenwärtigen Messungszeitpunkt größer als die im Voraus eingestellte Zeitperiode ist, Eintreten in einen Vier-Elektroden-Gemeinschaftsmodus und Fortfahren, den Schritt des Berechnens der gesamten Körperzusammensetzung des gegenwärtigen Benutzers basierend auf dem Gewicht des gegenwärtigen Benutzers, der ersten Impedanz und den erlangten Benutzerattributinformationen des gegenwärtigen Benutzers durchzuführen; oder
wenn bestimmt wird, dass das Zeitintervall zwischen dem Messungszeitpunkt, zu dem der Acht-Elektroden-Modus zuletzt verwendet wurde, und dem gegenwärtigen Messungszeitpunkt kleiner als die im Voraus eingestellte Zeitperiode ist, Eintreten in einen Vier-Elektroden-Expansionsmodus und Fortfahren, den Schritt des Berechnens der gesamten Körperzusammensetzung und der segmentalen Körperzusammensetzung des gegenwärtigen Benutzers basierend auf dem Gewicht des gegenwärtigen Benutzers, der ersten Impedanz, einer erlangten Impedanz außer der ersten Impedanz des gegenwärtigen Benutzers und den erlangten Benutzerattributinformationen des gegenwärtigen Benutzers durchzuführen.

2. Körperzusammensetzung-Detektionsgerät, umfassend Mittel zum:
Detektieren eines durch einen gegenwärtigen Benutzer verwendeten Detektionsmodus;
Durchführen einer Identitätsidentifizierung an dem gegenwärtigen Benutzer basierend auf dem Detektionsmodus, einem gemessenen Gewicht und mindestens einer Impedanz des gegenwärtigen Benutzers und erlangten Benutzerdaten einer Vielzahl von Benutzern, um ein Benutzeridentifizierungsergebnis zu erzeugen; und
Erzeugen einer Körperzusammensetzung des gegenwärtigen Benutzers in verschiedenen Detektionsmodi basierend auf erlangten Detektionsdaten in den verschiedenen Detektionsmodi;
wobei der Detektionsmodus einen Vier-Elektroden-Modus umfasst, die mindestens eine Impedanz eine erste Impedanz umfasst und die Benutzerdaten ein historisches Gewicht, eine historische erste Impedanz, eine historische Körperfettrate und Benutzerattributinformationen umfassen; und
das Durchführen einer Identitätsidentifizierung an dem gegenwärtigen Benutzer basierend auf dem Detektionsmodus, einem gemessenen Gewicht und mindestens einer Impedanz des gegenwärtigen Benutzers und erlangten Benutzerdaten einer Vielzahl von Benutzern, um ein Benutzeridentifizierungsergebnis zu erzeugen, umfasst:
Bestimmen eines ersten Benutzersatzes basierend auf dem gemessenen Gewicht des gegenwärtigen Benutzers und den historischen Gewichten der Vielzahl von Benutzern, die den gegenwärtigen Benutzer umfassen;
Bestimmen eines zweiten Benutzersatzes basierend auf dem ersten Benutzersatz, dem gemessenen Gewicht des gegenwärtigen Benutzers, der ersten Impedanz und der historischen ersten Impedanz, der historischen Körperfettrate und den Benutzerattributinformationen jedes Benutzers; und
Durchführen einer Identitätsidentifizierung an dem gegenwärtigen Benutzer basierend auf dem zweiten Benutzersatz, um das Benutzeridentifizierungsergebnis zu erzeugen; wobei das Durchführen einer Identitätsidentifizierung an dem gegenwärtigen Benutzer basierend auf dem zweiten Benutzersatz, um das Benutzeridentifizierungsergebnis zu erzeugen, umfasst:
Auswählen, aus dem zweiten Benutzersatz, eines Benutzers korrespondierend mit einer minimalen dritten Änderungsrate und Bestimmen des Benutzers korrespondierend mit der minimalen dritten Änderungsrate als den gegenwärtigen Benutzer;
wobei
nach dem Auswählen, aus dem zweiten Benutzersatz, eines Benutzers korrespondierend mit einer minimalen dritten Änderungsrate und Bestimmen des Benutzers korrespondierend mit der minimalen dritten Änderungsrate als den gegenwärtigen Benutzer das Gerät ferner umfasst:
Erlangen eines gegenwärtigen Messungszeitpunkts und Bestimmen, ob ein Zeitintervall zwischen einem Messungszeitpunkt, zu dem ein Acht-Elektroden-Modus zur Detektion zuletzt verwendet wurde, und dem gegenwärtigen Messungszeitpunkt kleiner als eine im Voraus eingestellte Zeitperiode ist; und
wenn bestimmt wird, dass das Zeitintervall zwischen dem Messungszeitpunkt, zu dem der Acht-Elektroden-Modus zuletzt verwendet wurde, und dem gegenwärtigen Messungszeitpunkt größer als die im Voraus eingestellte Zeitperiode ist, Eintreten in den Vier-Elektroden-Gemeinschaftsmodus und Fortfahren, den Schritt des Berechnens der gesamten Körperzusammensetzung des gegenwärtigen Benutzers basierend auf dem Gewicht des gegenwärtigen Benutzers, der ersten Impedanz und den erlangten Benutzerattributinformationen des gegenwärtigen Benutzers durchzuführen; oder
wenn bestimmt wird, dass das Zeitintervall zwischen dem Messungszeitpunkt, zu dem der Acht-Elektroden-Modus zuletzt verwendet wurde, und dem gegenwärtigen Messungszeitpunkt kleiner als die im Voraus eingestellte Zeitperiode ist, Eintreten in einen Vier-Elektroden-Erweiterungsmodus und Fortfahren, den Schritt des Berechnens der gesamten Körperzusammensetzung und der segmentalen Körperzusammensetzung des gegenwärtigen Benutzers basierend auf dem Gewicht des gegenwärtigen Benutzers, der ersten Impedanz, einer erlangten Impedanz außer der ersten Impedanz des gegenwärtigen Benutzers und den erlangten Benutzerattributinformationen des gegenwärtigen Benutzers durchzuführen.

## Revendications

1. Procédé de détection de composition corporelle, comprenant :
la détection (102) d'un mode de détection utilisé par un utilisateur actuel ;
la réalisation (110, 120) d'une identification d'identité sur l'utilisateur actuel basée sur le mode de détection, un poids mesuré et au moins une impédance de l'utilisateur actuel, et des données utilisateur obtenues d'une pluralité d'utilisateurs, pour générer un résultat d'identification d'utilisateur ; et
la génération de composition corporelle de l'utilisateur actuel dans différents modes de détection basée sur des données de détection obtenues dans les différents modes de détection,
le mode de détection comprenant un mode à quatre électrodes, l'au moins une impédance comprenant une première impédance, et les données utilisateur comprenant un poids historique, une première impédance historique, un taux de graisse corporelle historique, et des informations d'attribut utilisateur ; et
la réalisation d'une identification d'identité sur l'utilisateur actuel basée sur le mode de détection, un poids mesuré et au moins une impédance de l'utilisateur actuel, et des données utilisateur obtenues d'une pluralité d'utilisateurs, pour générer un résultat d'identification d'utilisateur comprenant :
la détermination d'un premier ensemble d'utilisateurs basée sur le poids mesuré de l'utilisateur actuel et les poids historiques de la pluralité d'utilisateurs qui comprennent l'utilisateur actuel ;
la détermination d'un deuxième ensemble d'utilisateurs basée sur le premier ensemble d'utilisateurs, le poids mesuré de l'utilisateur actuel, la première impédance, et la première impédance historique, le taux de graisse corporelle historique, et les informations d'attribut utilisateur de chaque utilisateur ; et
la réalisation d'une identification d'identité sur l'utilisateur actuel basée sur le deuxième ensemble d'utilisateurs, pour générer le résultat d'identification d'utilisateur ;
la réalisation d'une identification d'identité sur l'utilisateur actuel basée sur le deuxième ensemble d'utilisateurs, pour générer le résultat d'identification d'utilisateur comprenant :
la sélection, à partir du deuxième ensemble d'utilisateurs, d'un utilisateur correspondant à un troisième taux de changement minimal, et la détermination de l'utilisateur correspondant au troisième taux de changement minimal comme étant l'utilisateur actuel ;
après la sélection, à partir du deuxième ensemble d'utilisateurs, d'un utilisateur correspondant à un troisième taux de changement minimal, et la détermination de l'utilisateur correspondant au troisième taux de changement minimal comme étant l'utilisateur actuel, le procédé comprenant en outre :
l'obtention d'un point temporel de mesure actuel, et la détermination si un intervalle de temps entre un point temporel de mesure auquel un mode à huit électrodes est utilisé pour la dernière détection et le point temporel de mesure actuel est inférieur à une période de temps prédéfinie ; et
s'il est déterminé que l'intervalle de temps entre le point temporel de mesure auquel le mode à huit électrodes est utilisé la dernière fois et le point temporel de mesure actuel est supérieur à la période de temps prédéfinie, l'entrée dans un mode commun à quatre électrodes, et la poursuite de l'exécution de l'étape de calcul de la composition corporelle totale de l'utilisateur actuel basée sur le poids de l'utilisateur actuel, la première impédance, et les informations d'attribut utilisateur obtenues de l'utilisateur actuel ; ou
s'il est déterminé que l'intervalle de temps entre le point temporel de mesure auquel le mode à huit électrodes est utilisé la dernière fois et le point temporel de mesure actuel est inférieur à la période de temps prédéfinie, l'entrée dans un mode d'expansion à quatre électrodes, et la poursuite de l'exécution de l'étape de calcul de la composition corporelle totale et de la composition corporelle segmentaire de l'utilisateur actuel basée sur le poids de l'utilisateur actuel, la première impédance, une impédance obtenue autre que la première impédance de l'utilisateur actuel, et les informations d'attribut utilisateur obtenues de l'utilisateur actuel.

2. Dispositif de détection de composition corporelle, comprenant des moyens pour :
la détection d'un mode de détection utilisé par un utilisateur actuel ;
la réalisation d'une identification d'identité sur l'utilisateur actuel basée sur le mode de détection, un poids mesuré et au moins une impédance de l'utilisateur actuel, et des données utilisateur obtenues d'une pluralité d'utilisateurs, pour générer un résultat d'identification d'utilisateur ; et
la génération de composition corporelle de l'utilisateur actuel dans différents modes de détection basée sur des données de détection obtenues dans les différents modes de détection ;
le mode de détection comprenant un mode à quatre électrodes, l'au moins une impédance comprenant une première impédance, et les données utilisateur comprenant un poids historique, une première impédance historique, un taux de graisse corporelle historique, et des informations d'attribut utilisateur ; et
la réalisation d'une identification d'identité sur l'utilisateur actuel basée sur le mode de détection, un poids mesuré et au moins une impédance de l'utilisateur actuel, et des données utilisateur obtenues d'une pluralité d'utilisateurs, pour générer un résultat d'identification d'utilisateur comprenant :
la détermination d'un premier ensemble d'utilisateurs basée sur le poids mesuré de l'utilisateur actuel et les poids historiques de la pluralité d'utilisateurs qui comprennent l'utilisateur actuel ;
la détermination d'un deuxième ensemble d'utilisateurs basée sur le premier ensemble d'utilisateurs, le poids mesuré de l'utilisateur actuel, la première impédance et la première impédance historique, le taux de graisse corporelle historique, et les informations d'attribut utilisateur de chaque utilisateur ; et
la réalisation d'une identification d'identité sur l'utilisateur actuel basée sur le deuxième ensemble d'utilisateurs pour générer le résultat d'identification d'utilisateur ;
la réalisation d'une identification d'identité sur l'utilisateur actuel basée sur le deuxième ensemble d'utilisateurs, pour générer le résultat d'identification d'utilisateur comprenant :
la sélection, à partir du deuxième ensemble d'utilisateurs, d'un utilisateur correspondant à un troisième taux de changement minimal, et la détermination de l'utilisateur correspondant au troisième taux de changement minimal comme étant l'utilisateur actuel ;
après la sélection, à partir du deuxième ensemble d'utilisateurs, d'un utilisateur correspondant à un troisième taux de changement minimal, et la détermination de l'utilisateur correspondant au troisième taux de changement minimal comme étant l'utilisateur actuel, le dispositif comprenant en outre :
l'obtention d'un point temporel de mesure actuel, et la détermination si un intervalle de temps entre un point temporel de mesure auquel un mode à huit électrodes est utilisé pour la dernière détection et le point temporel de mesure actuel est inférieur à une période de temps prédéfinie ; et
s'il est déterminé que l'intervalle de temps entre le point temporel de mesure auquel le mode à huit électrodes est utilisé la dernière fois et le point temporel de mesure actuel est supérieur à la période de temps prédéfinie, l'entrée dans le mode commun à quatre électrodes, et la poursuite de l'exécution de l'étape de calcul de la composition corporelle totale de l'utilisateur actuel basée sur le poids de l'utilisateur actuel, la première impédance, et les informations d'attribut utilisateur obtenues de l'utilisateur actuel ; ou
s'il est déterminé que l'intervalle de temps entre le point temporel de mesure auquel le mode à huit électrodes est utilisé la dernière fois et le point temporel de mesure actuel est inférieur à la période de temps prédéfinie, l'entrée dans un mode d'expansion à quatre électrodes, et la poursuite de l'exécution de l'étape de calcul de la composition corporelle totale et de la composition corporelle segmentaire de l'utilisateur actuel basée sur le poids de l'utilisateur actuel, la première impédance, une impédance obtenue autre que la première impédance de l'utilisateur actuel, et les informations d'attribut utilisateur obtenues de l'utilisateur actuel.
